# EUROPEAN PATENT SPECIFICATION

(11) **EP 1 575 572 B1**
(45) Date of publication and mention of the grant of the patent: **24.09.2014**
(21) Application number: 03745621.7
(22) Date of filing: 26.03.2003
(51) Int. Cl.: C12N 5/00, A61L 27/36, A61K 35/50

(54) **COLLAGEN BIOFABRIC AND METHODS OF PREPARATION AND USE THEREFOR**
BIOLOGISCHES KOLLAGENGEWEBE UND HERSTELLUNGS- UND VERWENDUNGSVERFAHREN DAFÜR
TEXTURE BIOLOGIQUE COLLAGENE ET PROCEDES PERMETTANT DE LA PREPARER ET DE L'UTILISER

(30) Priority: 26.03.2002 US 106653
(43) Date of publication of application: 21.09.2005
(60) Divisional application: 13181876.7
(73) Proprietor: Anthrogenesis Corporation, Warren NJ 07059 (US)
(72) Inventor: HARIRI, Robert, J., Florham Park, NJ 07932 (US); KAPLUNOVSKY, Aleksandr, M., Rockaway, NJ 07866 (US); MURPHY, Patricia, A., Wayne, NJ 07470 (US)
(74) Representative: Jones Day
(86) International application number: PCT/US2003/009304
(87) International publication number: WO 2003/082201

(56) References cited:
- EP-A2- 0 518 389
- US-A- 4 446 234
- US-A- 5 612 028
- US-A- 5 632 778
- US-A- 6 152 142
- US-A- 6 152 142
- US-B1- 6 326 019
- LIOTTA L A ET AL: "New method for preparing large surfaces of intact human basement membrane for tumor invasion studies" CANCER LETTERS, NEW YORK, NY, US LNKD- DOI:10.1016/0304-3835(80)90105-6, vol. 11, no. 2, 1 December 1980 (1980-12-01), pages 141-152, XP026176880 ISSN: 0304-3835 [retrieved on 1980-12-01]

## Description

### 1. FIELD OF THE INVENTION

The present invention relates to a method of preparing an amniotic membrane laminate from a placenta having an amniotic membrane and a chorionic membrane. The present invention further relates to an amniotic membrane laminate obtainable by the method of the present invention.

The present invention comprises collagenous membranes produced from amnion, herein referred to as a collagen biofabric. The collagen biofabric comprised in the invention has the structural integrity of the native non-treated amniotic membrane, *i.e*., the native tertiary and quaternary structure. The present disclosure provides a method for preparing a collagen biofabric from a placental membrane, preferably a human placental membrane having a chorionic and amniotic membrane, by decellularizing the amniotic membrane. In a preferred embodiment, the amniotic membrane is completely decellularized. The collagen biofabric comprised in the invention has numerous utilities in the medical and surgical field including for example, blood vessel repair, construction and replacement of a blood vessel, tendon and ligament replacement, wound-dressing, surgical grafts, ophthalmic uses, sutures, and others. The benefits of the biofabric are, in part, due to its physical properties such as biomechanical strength, flexibility, suturability, and low immunogenicity, particularly when derived from human placenta.

### 2. BACKGROUND OF THE INVENTION

### 2.1 AMNIOTIC MEMBRANE: ANATOMY AND HISTOLOGY

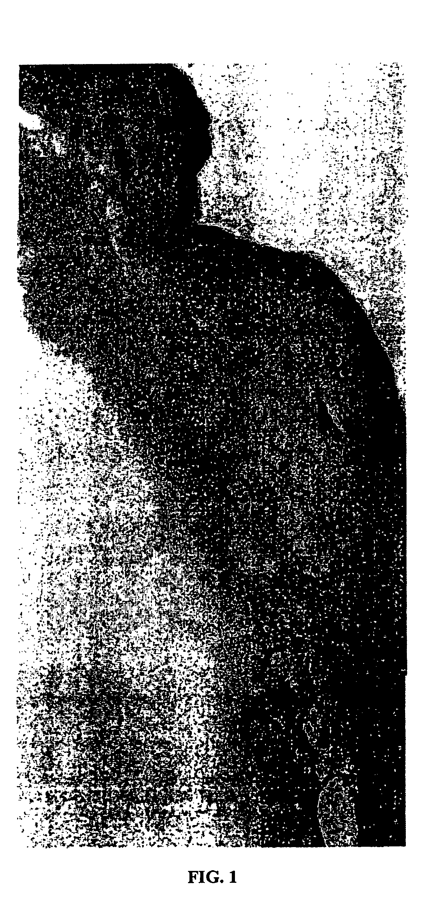

The placental sac is composed of two layers intimately connected by loose connective tissue. They are known as the amniotic and chorionic layers *(See* FIG. 1). The amniotic layer is the most internal of the two layers and comes into contact with the amniotic fluid that surrounds the fetus and together they form the amniotic sac. The amniotic layer is avascular and lined by simple columnar epithelium overlying a basal membrane and it measures 30-60 microns in thickness. The chorionic membrane is the outer layer of the sac and it is heavily cellularized. The vascular tree originates in the placenta and extends to the placental membranes through the chorionic layer. The chorionic layer is separated from the amniotic layer by loose connective tissue and combined, the two layers measure 120-180 microns. The placental membranes have a collagen matrix that is heavily laden with mucopolysaccarides and they are believed to serve primarily as a protective sac for the developing fetus. The membranes also maintain a barrier for infectious and immnunologic agents present in the maternal circulation. Placental membranes have both active and passive transports. Most small molecules and proteins can travel freely through them but large proteins such as IgM cannot cross through the basal layer.

Preservation of the placental membranes (which are either from animal or human sources) in either 95% ethyl alcohol or glycerol mixed in 50-50% proportions with tissue culture media has been utilized for preservation of the amniotic membrane prior to freezing. These preservatives eliminate the vitality of the placental tissues making the nuclei pyknotic but the collagen matrix and basal membranes are preserved. Interestingly, both forms of preservation also eliminate the antigenicity of the transplanted membranes and also any potentially virulent agents. Preservation is usually accomplished after the amniotic membranes are carefully separated from the chorion. The side of the amniotic membrane with the basal lamina and epithelium is shiny and the opposite side facing the chorion is dull.

### 2.2 PREVIOUS CLINICAL APPLICATIONS OF AMNIOTIC MEMBRANES

Possible potential problems with xenogenic tissues (tissues from other species) carrying zoonotic diseases or causing cross-species rejection have made these tissues less desirable. Allogenic grafts, or grafts from different individuals of the same species, continue to be the preferred source for human graft materials. The scarcity of human donor tissues for grafting is a growing problem that has stimulated the development of new materials for tissue grafting. Most often these sources of biological raw material are scarce, difficult to obtain, and very costly. The collagen sheet from human amnion, however, has desirable biomechanical characteristics useful in tissue graft applications. Thus, amniotic membranes are a good source of allogenic graft material.

The fetal membrane including the amniotic and chorionic membrane has been used in surgeries documented as early as 1910 and has been reviewed by Trelford and Trelford-Sauder in 1979 (*See* Trelford and Trelford-Sauder, 1979, Am. J. Obstet. Gynecol. 833). In 1910 Davis was the first to report the use of fetal membranes as a surgical material in skin transplantation for example on burned and ulcerated skins (Davis et al., 1910, Johns Hopkins Med. J.; 15: 307). These studies were mainly in animals and human trials proved disappointing. Since then the use of amniotic membranes in surgery has been expanded *(See, e.g.,* Stem et al., 1913, JAMA, 13: 973-4; Sabella et al., 1913 Med. Records NY, 83: 478-80; de Rotth et al., 1940 Arch. Opthalmol, 23: 522-5; Sorsby et al., 1946, Br. J. Opthamlol. 30: 337-45). It is now utilized as a biological dressing for burned skin, skin wounds, and chronic ulcers of the leg, as an adjunctive tissue in surgical reconstruction of artificial vagina, and for repairing omphaloceles *(See e.g.,* Trelford and Trelford-Sauder, 1979, 134 Am. J. Obstet. Gynecol. 833; Colocho et al., 1974 Arch. Surg. 109: 370-3; Faulk et al. 1980, Lancet, 1156; Prasad et al., 1986, J. Trauma, 26: 945-6; Subrahmanyan et al., 1995, J. Plastic Surg. 48: 477-8; Gruss et al., 1978, J. Can. Med. Assoc. 118: 1237-46; Ward et al., 1984, Br. J. Plastic Surg. 37: 191-3; Dhall, 1984, J. Obstet. Gynaecol. 91: 279-82). It has also been used to prevent tissue adhesion in surgical procedures of the abdomen, head and pelvis *(*Gharib et al., 1996, Pediatr. Surg. Int. 11: 96-9; Rennekampff et al., 1994, Invest. Surg. 7: 187-93). In the 1940s, several authors reported the beneficial role of the amniotic membrane in treating a variety of ocular surface disorders *(See e.g.,* de Rotth et al., 1940 Arch. Opthalmol, 23: 522-5; Sorsby et al., 1946, Br. J. Opthalmol. 30: 337-45; Lavery et al., 1946, Trans. Opthalmol. Soc. UK, 66: 668).

Numerous attempts in the field to optimize the preparation and preservation of the amniotic membranes for use in transplantation have been previously described *(see e.g.,* Dua et al., 1999, Br. J. Opthalmol. 83: 748-52 ("Dua") for a review). Various preparation of amniotic membranes have included preservation by saline and antibiotic mixtures, alcohol dehydration with or without separation of the amniotic layer from the chorionic layer. However, all of the methods described in Dua, and in the references described above still carry shortcomings that need to be addressed by improvements in preparation and preservation of amniotic membranes.

More recently, methods have been disclosed which rely on freezing for preservation of the amniotic membrane for application in tissue graft surgical procedures to correct corneal epithelial defects. *See e.g*., U.S. Patent Nos. 6,152,142 and 6,326,019B1 ("Tseng"). Tseng discloses an amniotic membrane that is mounted on a substrate and preserved in a mixture of Dulbecco's Modified Eagle Medium and glycerol and frozen at-80°C. The process of freezing the tissue at any time during its preparation makes the Tseng amniotic membrane brittle, and even more brittle after the steps of thawing and activation. In addition, the thawing and activation steps add time required for the handling of the amniotic membrane. Furthermore, because of the brittleness of the Tseng amniotic membrane caused by the freezing step in the preservation and preparation process, a structural support or backing is required to ensure structural integrity of the Tseng amniotic membrane during storage. This presents the added difficulty of separating the preserved amniotic membrane from the backing, which, due to its brittleness can be difficult to handle and separate intact. Separation of the amnion membrane from the backing thus increases the likelihood of rupture of the membrane, and increases the length of time required to activate the amniotic membrane to allow for thorough impregnation of the activation solution into the frozen amniotic membrane prior to performing the surgical procedure, leading to increased preparation time in the surgical suite. Storage and shipping are also complicated by the requirement of -80°C freezing. Finally, the membranes of Tseng are not generally decellularized; as a result, the amniotic membranes so prepared are typically opaque and do not have a uniform structural composition.

More recently, Yui et al., U.S. Patent Nos. 5,618,312, (the "'312 Patent") described the preparation of collagen sheets from stratum compactum of tissue membrane. Although the material described is primarily collagen, it is weak enough due to its processing to require a separate step of crosslinking in order to render it strong enough for medical use, e.g., suturable. One method for cross-linking described in the '312 Patent employs high heat treatment, preferably at 100° to 110°C, which is believed to adversely affect the native conformation of collagen. Yui et al., U.S. Patent No. 5,876,451 describe a similar collagen material derived from placenta. This material, however, is treated during preparation with proteases as part of a decellularization step, which likely results in destruction and/or disruption of native conformation of the components of the matrix; thus, the resulting collagen matrix does not maintain its native conformation.

There thus remains a need in the art for an improved amniotic membrane for use in medical, diagnostics, and cosmetic applications, which has improved structural characteristics. The present invention provides such a biofabric, comprising collagen that, unlike prior collagen-based biofabrics, retains the native collagen quaternary structure. As a result, the biofabric of the present invention is easily prepared, easily used, and is strong enough for medical and surgical purposes, and provides a superior substrate for wound healing.

### 3. SUMMARY OF THE INVENTION

The present invention relates to a method of preparing an amniotic membrane laminate from a placenta having an amniotic membrane and a chorionic membrane comprising: (a) separating the amniotic membrane from the chorionic membrane; (b) decellularizing the amniotic membrane; and (c) layering at least two of the decellularized amniotic membranes in contact with each other so that an amniotic membrane laminate is formed, wherein the decellularization of the amniotic membrane does not constitute freezing at any point in preparation of the amniotic membrane or contacting the membrane with protease.

Further, the present invention relates to an amniotic membrane laminate comprising at least two layers of a dehydrated, decellularized and substratefree amniotic membrane, wherein said amniotic membrane has a native tertiary and quaternary structure.

The present invention relates, in part, to the discovery by the inventors of a novel method of preparation of a collagen biofabric from placenta, preferably a human placenta, that results in a novel collagen biofabric with improved physical and biophysical properties. Preferably the method of preparation involves minimal manipulation of the amniotic membrane. The collagen biofabric comprised in the invention unlike those described in the prior art, due to the way by which it is processed, has an intact native tertiary and quaternary structure. The present invention also provides a placental-derived amniotic membrane or biofabric having superior characteristics of increased tensile strength, suturability, and reduced immunogenicity resulting in reduced host-graft rejection. The present invention also provides a placental-derived amniotic membrane or biofabric that can be stored as dehydrated sheets without freezing or cryopreservation. Preferably, the placental-derived amniotic membrane is derived from a human placenta for use in human patients. However, the same methods can be employed using placentas from various animal species for veterinary use in animal patients.

The present disclosure provides a method of preparing a collagen biofabric comprising providing a placenta, preferably a human placenta, separating the amnionic and chorionic layers from each other, and decellularizing the amniotic membrane while preserving the architecture of the underlying extracellular matrix. The method further entails washing and drying the decellularized membrane. This method yields a dehydrated, decellularized biofabric that can remain stable under sterile storage conditions at room temperature and that is subsequently rehydrated and grafted to or implanted into a subject.

In a specific embodiment, the placenta is from a human female who has undergone a cesarean section delivery or natural delivery. In preferred embodiments, common seriological and bacteriological assays known to one skilled in the art are used to determine if the placenta is from a donor that is free of a communicable disease. In a specific embodiment, the placenta has been tested to be free of at least one communicable disease. In another embodiment, the source of the placenta is known including medical history, blood type, immunologic data, and genotype characteristics of the donor. Although, the placenta can be from any mammal, preferably the donor is a human female. In some embodiments, the placenta is exsanguinated using common methods known to one skilled in the art prior to separating the amniotic membrane from the chorionic membrane.

The decellularization of the amniotic membrane in accordance with the methods of the invention comprises removing all visible cellular material and cellular debris from the amniotic membrane, e.g., from the maternal side of the amniotic membrane and the fetal side of the amniotic membrane. The decellularization of the amniotic membrane should not result in disruption of the structural integrity of the amniotic membrane or alter the biochemical composition. Accordingly, decellularizing the amniotic membrane in accordance with the methods of the invention does not constitute freezing at any point in preparation of the amniotic membrane or contacting the membrane with a protease. Preferably, the amniotic membrane is decelluarized using a weak detergent containing solution, e.g., a solution comprising 0.01-1.0% deoxycholic acid sodium salt monohydrate, a nonionic detergents, Triton X-100, an anionic detergent, or sodium dodecyl sulfate or a combination thereof.

Once the amniotic membrane is decellularized in accordance with the methods of the invention, the membrane may be washed and dried, preferably with low heat under vacuum.

In some embodiments, the invention comprises a collagen biofabric comprising a dehydrated, decellularized and substrate-free amniotic membrane, so that the membrane has native tertiary and quaternary structure. In other embodiments, the invention comprises a collagen biofabric comprising a decellularized substrate-free amniotic membrane, comprising of collagen, elastin and fibronectin. In yet other embodiments, the invention comprises a collagen biofabric comprising a dehydrated, decellularized, uniform, translucent, and substrate-free amniotic membrane, with the provision that the amniotic membrane has never been contacted with a protease.

In some embodiments, the biofabric further comprises one or more biomolecules, *e.g*., therapeutic agents, including but not limited to, antibiotics, hormones, growth factors, anti-tumor agents, anti-fungal agents, anti-viral agents, pain medications, anti-histamines, anti-inflammatory agents, anti-infectives, wound healing agents, wound sealants, cellular attractants and scaffolding reagents, and the like. In a specific example, the collagen biofabric may be impregnated with one or more growth factors, for example, fibroblast growth factor, epithelial growth factor, *etc.* The biofabric may also be impregnated with one or more small molecules, including but not limited to small organic molecules such as specific inhibitors of particular biochemical processes *e.g*., membrane receptor inhibitors, kinase inhibitors, growth inhibitors, anti-cancer drugs, antibiotics, *etc.* In some embodiments, the collagen biofabric is impregnated with a biomolecule, during production or during preparation for surgery depending on its intended use.

In some embodiments, the invention encompasses a laminate comprising at least two layers of the biofabric of the invention, and methods of preparing same. In other embodiments, the invention encompasses shaping the laminates into complex three dimensional scaffolds depending on the intended use, including but not limited to sheets, fibers, spheres, tubes.

In one embodiment, the invention encompasses a method of preparing an amniotic membrane laminate comprising: providing a placenta comprising an amniotic membrane and a chorionic membrane; separating the amniotic membrane from the chorionic membrane; and decellularizing the amniotic membrane. In another embodiment, the method further comprises washing the decellularized amniotic membrane at least once; layering at least two of the decellularized amniotic membranes in contact with each other so that an amniotic membrane laminate is formed; and drying the decellularized amniotic membrane laminate. Alternatively, in another embodiment, the method for preparing an amniotic membrane laminate comprises, drying at least two amniotic membranes prepared in accordance with the methods of the invention, and layering the at least two amniotic membranes in contact with each other so that an amniotic membrane laminate is formed.

In some embodiments, the amniotic membrane layers produced in accordance with the methods of the invention may be placed in contact with each other in the presence of an adhesive to form an amniotic membrane laminate. The adhesive used in accordance with the methods and compositions of the invention may be any biological glue known to one skilled in the art, preferably a biocompatible glue, including but not limited to, natural glue, e.g., fibronectin, fibrin, synthetic glue. In other embodiments, the amniotic membrane layers prepared in accordance to the methods of the invention are cross-linked to each other to form an amniotic membrane laminate. Any cross-linking reagent and method known to one skilled in the art is within the scope of the present invention, including but not limited to, chemical cross-linking, peptide cross-linking, UV cross-linking, radiation cross-linking, fibronectin cross-linking, fibrinogen cross-linking, hydrogel cross-linking. In other embodiments, the amniotic membrane laminates produced in accordance with the methods of the invention do not comprise an adhesive.

For reference only, the present disclosure encompasses using the collagen biofabric as a surgical graft or the use of the surgical graft in a surgical procedure in a subject, preferably a human, comprising placing the graft directly on the surgical site.

The invention provides an amniotic membrane laminate, or a three-dimensional scaffold further comprising one or more hydrogel compositions, and methods of preparing same. The hydrogel composition may comprise a polymer inlcuding but not limited to polyvinyl alcohol, polyethylene glycol, hyaluronic acid, and derivative and analogs thereof.

In some embodiments, the collagen biofabric comprised in the invention, aminates, three-dimensional scaffolds, or hydrogel compositions thereof may be further populated with cells, such as stem cells, differentiated adult cells, progenitor cells, and the like, preferably human so that the cells are uniform and confluent. The cells are not of human embryonic origin.

Disclosed herein are methods of high level production of the collagen biofabric, laminates thereof, three-dimensional scaffolds thereof, and hydrogel compositions thereof, particularly but not limited to, commercial scale production. The invention solves difficulties in producing large-scale quantities of amniotic membranes for use in clinical trials and commercial sales.

Disclosed herein are compositions comprising a collagen biofabric disclosed herein suitable for drug delivery; tissue engineering; urological related uses, *e.g*., correction of urinary incontinence; ocular uses, *e*.*g*.*,* for the treatment of an ocular surface disorder, and as an ophthalmic surgical graft; vascular uses, e.g., blood vessel repair, construction and replacement of a blood vessel; cardiological uses, e.g., as a prosthetic device in constructing diseased valves; neuronal-related uses, e.g., repair of injured nerves, especially severed peripheral nerves, as a dural substitute, and as a prostheses around nerve anastosmosis; bone related uses, e.g., for the treatment of orthopedic defects, as a bone replacement; dermatological uses, *e.g.,* for the treatment of wounds (external and internal), acute and chronic wounds, congenital wounds, and burns; for the treatment of skin conditions, e.g., skin lesions, aged skin, wrinkles, fine lines, thinning, reduced skin elasticity, rough skin, and sun damaged skin; as a wound dressing; and for the treatment of wound infections.

Disclosed herein is a method for treating and/or preventing an eye related disease or disorder, e.g., ocular surface disease, in a subject, disclosed herein comprising using the collagen biofabric disclosed herein for example, by placing the biofabric as a surgical graft on the diseased corneal surface of the subject or a method of treating and/or preventing a skin condition in a subject, comprising using a biofabric of the invention for example, by contacting the skin with the biofabric, or treating a wound and/or burn in a subject comprising contacting the wound and/or burn with a biofabric of the invention, or a method of correcting urinary incontinence in a subject, preferably, a human, comprising using a collagen biofabric as an implant.

Disclosed herein is a method of delivering a therapeutic agent to a subject comprising contacting the subject with a collagen biofabric disclosed herein, or methods of delivering cells to a subject comprising populating a collagen biofabric or laminate of the invention with living cells for example in tissue engineering.

Disclosed herein is a surgical graft comprising the biofabric of the invention for use in a surgical procedure. The surgical graft may be applied to an internal or external site of the subject, preferably a human.

### 4. BRIEF DESCRIPTION OF THE FIGURES

**FIG. 1** The chorion and amniotic membrane of a human placenta.
**FIG. 2** **PHOTOMICROGRAPH OF THE COLLAGEN BIOFABRIC**

### A. BEFORE PROCESSING

### B. AFTER PROCESSING

**FIG. 3** **THE COLLAGEN BIOFABRIC**. The collagen biofabric comprised in the invention is exemplified having a uniform translucent surface with an embossed pattern.

FIG.4 MESH FRAME **AND THE** BIOFABRIC BEING DRIED THEREIN

### 5. DETAILED DESCRIPTION OF THE INVENTION

The present invention relates to a method of preparing an amniotic membrane laminate from a placenta having an amniotic membrane and a chorionic membrane comprising: (a) separating the amniotic membrane from the chorionic membrane; (b) decellularizing the amniotic membrane; and (c) layering at least two of the decellularized amniotic membranes in contact with each other so that an amniotic membrane laminate is formed, wherein the decellularization of the amniotic membrane does not constitute freezing at any point in preparation of the amniotic membrane or contacting the membrane with protease.

Further, the present invention relates to an amniotic membrane laminate comprising at least two layers of a dehydrated, decellularized and substratefree amniotic membrane, wherein said amniotic membrane has a native tertiary and quaternary structure.

The present invention provides a collageneous membrane or biofabric derived from the placenta of a mammal, preferably of a human. The collagen biofabric is prepared so as to retain the native collagen conformation, *i.e.,* the native tertiary and quaternary conformation, in the final product. In addition to the collagen biofabric, disclosed herein are methods of making the collagen biofabric, and of using the biofabric in a medical setting.

The present invention comprises a collagen biofabric comprising a dehydrated, decellularized and substrate-free amniotic membrane so that the amniotic membrane has a native tertiary and quaternary structure. In some embodiments the invention comprises a decellularized and substrate-free collagen biofabric comprising of collagen, elastin, and fibronectin.

In some embodiments, the invention provides an amniotic membrane laminate comprising a collagen biofabric as disclosed herein. The amniotic membrane laminate prepared in accordance with the methods of the invention comprises at least two layers of the collagen biofabric that are placed in contact with each other to form the amniotic membrane laminate. In other embodiments, the invention comprises a three dimensional scaffold, such as a tube, comprising a collagen biofabric as disclosed herein. In yet other embodiments, the invention comprises a collagen biofabric as disclosed herein, a laminate thereof, or a three dimensional scaffold thereof further comprising a hydrogel composition.

The invention thus comprises various forms and configurations of the collagen biofabric including but not limited to laminates, three-dimensional scaffolds, and hydrogel compositions. Disclosed herein is/are any medical useful form of the compositions of the invention. Regardless of the particular form or configuration, the compositions of the invention may further comprise one or more biomolecules, preferably a therapeutic agent. The compositions of the invention comprising a biomolecule have numerous utility in the medical field as described in detail herein. In some embodiments, the invention encompasses populating the compositions of the invention with living cells so that the cells are uniform and confluent. The compositions of the invention populated with cells have numerous utility in the medical and dental field for example for tissue engineering purposes.

Disclosed herein is/are methods for preparing a collagen biofabric, a laminate thereof, a three-dimensional scaffold thereof, or a hydrogel composition. Disclosed herein is/are a method of preparing a collagen biofabric from a placenta having an amniotic membrane and a chorionic membrane comprising: separating the amniotic membrane from the chorionic membrane; and decellularizing the amniotic membrane so that the amniotic membrane is not contacted with an enzyme, e.g., a protease. The method further entails washing and drying the decellularized amniotic membrane. In another specific embodiment, the invention provides a method of preparing an amniotic membrane laminate from a placenta having an amniotic membrane and a chorionic membrane comprising: separating the amniotic membrane from the chorionic membrane; decellularizing the amniotic membrane; and layering at least two of the decellularized amniotic membranes in contact with each other so that an amniotic membrane laminate is formed. In a specific embodiment, the decellularized amniotic membrane is dried prior to layer. In another specific embodiment, the decellularized amniotic membrane is dried after is layered so that at least two of the decellularized amniotic membranes are in contact with each other.

Disclosed herein is/are a method of using the collagen biofabric of the invention, laminates thereof, three-dimensional scaffolds thereof, or hyrdogel compositions thereof in a medical, dental and surgical setting. In fact, it is expected that the compositions of the invention have an enhanced therapeutic and clinical utility relative to the other biomaterials known in the art. Disclosed herein is/are a method of treating and/or preventing an eye related disease or disorder in a subject using a composition of the invention, or a method of treating and/or preventing an eye related disease or disorder in a subject comprising placing the biofabric on a diseased eye surface of the subject.

Disclosed herein is/are a method of treating and/or preventing a skin condition in a subject, preferably a human, using a composition of the invention. The method comprises contacting the skin of the subject with the composition. Alternatively, the composition is placed directly on the surface of the skin of the subject, which is the site of the skin condition.

Disclosed herein is/are a method for treating a wound or a burn in a subject, preferably a human, comprising contacting the composition at the site of the wound or burn.

Disclosed herein is/are is the use of a composition of the invention (e.g., a collagen biofabric, an amniotic membrane laminate) in a surgical procedure, such as ophthalmic surgery; cardiovascular surgery; periodontal surgery; neurological surgery, dental surgery, and orthopedic surgery.

Disclosed herein is/are a method for using a composition of the invention for delivering a biomolecule, preferably a therapeutic agent to a subject, preferably a human. Disclosed herein is/are a method of delivering cells using a composition of the invention to a subject, preferably a human, wherein the composition has been further populated with cells.

### 5.1 COLLAGEN BIOFABRIC

Disclosed herein is/are a collagenous amniotic membrane, herein referred to as a collagen biofabric. The collagen biofabric maintains the structural integrity of the native, non-treated amniotic membrane, *i.e*., retains the tertiary and quaternary structure of the structural proteins in its compositions such as collagen, elastin, and possibly fibronectin. Thus, the collagen biofabric disclosed herein is composed of the same structural proteins as the native or non-treated amniotic membranes. Prior art methods of producing amniotic membranes require the use of proteases or high heat treatment, as a result these membranes do not maintain the tertiary and quaternary structure of the structural proteins in their composition.

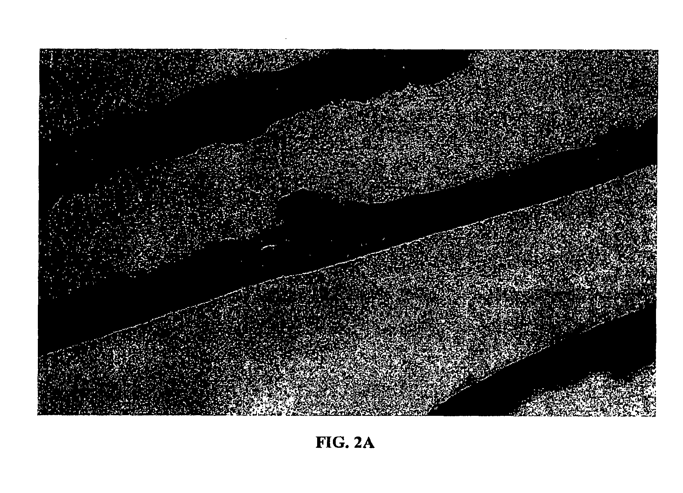

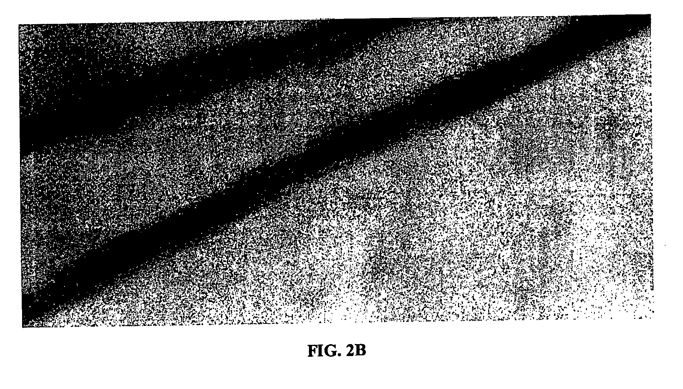

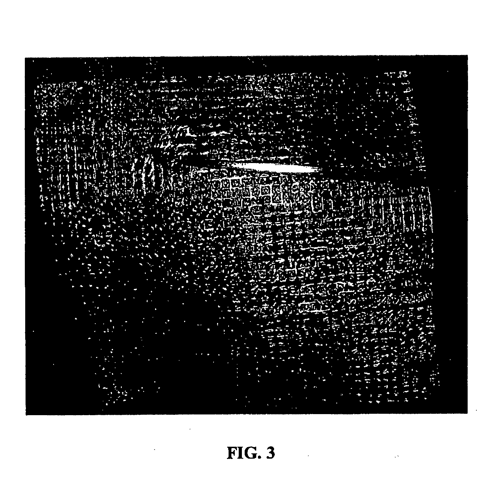

In a specific embodiment, the present invention comprises a dehydrated, decellularized, and substrate-free (*i.e.,* no filter backing) amniotic membrane such that the amniotic membrane has a native tertiary and quaternary structure *(See* FIG. 2A and B). The dehydrated decellularized amniotic membrane comprised in the invention is a uniform, *i.e.,* minimal to no cellular material, translucent biofabric, having an appearance as shown in FIG. 3 comprising a left handed triple helix alpha helical sheet of decellularized matrix *(See, e.g.,* Molecular Biology of the Cell, 1989, Alberts et al., ed., Garland Publishing Inc., New York, NY.

The collagen biofabric comprised in the invention may be derived from the amniotic membrane of any mammal, for example, equine, bovine, porcine or catarrhine sources, but is most preferably derived from human placenta. In a preferred embodiment, the biofabric of the invention has the native tertiary and quarternary structure of the collagenous material in its composition.

The collagen biofabric comprised in the invention in contrast to those described in the prior art is minimally manipulated, *i.e*., the collagen biofabric comprised in the invention is subjected to at most one chemical or biological treatment or manipulation, e.g., decellularization in a weak detergent. As used herein, "minimally manipulated" refers to a lack of enzymatic treatment, e.g., protease treatment, high heat treatment, harsh chemical treatment, exposure to strong detergents or acids of the amniotic membrane comprised in the invention at any step during the preparation of the collagen biofabric. Protease treatment of the amniotic membrane, as part of a decellularization step, compromises the structural integrity of the biofabric, e.g., affects the tertiary and/or quarternary structure of the collagen material. The minimal manipulation of the amniotic membrane in preparation of the biofabric of the invention, in contrast, results in a product with enhanced mechanical strength and a translucent product relative to those in the prior art.

The decellularized, substrate-free collagen biofabric comprised in the invention comprises of collagen (including, but not limited to, collagen type I, IV, and II), as well as fibronectin and elastin. This combination is in part responsible for the enhanced mechanical strength of the biofabric comprised in the invention, as prepared in accordance with the methods described herein. As a result of minimal processing and manipulation, the collagen biofabric comprised in the invention retains the composition of the native membrane. Collagen is the primary structural material of vertebrates and it is present in tissues of primarily mechanical function. The collagen molecule consists of three polypeptide chains called alpha chains (each about 1000 amino acids in length) twined around one another as in a three stranded rope forming a regular left-handed superhelix. At least 19 types of collagen have been identified and they all possess the same three-dimensional organization *(See, e.g.,* Lee et al., 2001, International J. of Pharmaceutics, 221: 1-22).

The amniotic membrane comprised in the present invention is superior in form, biomechanical, and structural features to those reported in the prior art and known to the inventors, in part, based on the discovery of a novel method of preparation of the amniotic membrane and a controlled and abundant source of human placenta, as described herein *(See* Section 5.2).

In particular, the collagen biofabric comprised in the present invention has one or more of the following characteristics as compared to one or more of the amniotic membranes of the prior art: enhanced tensile strength; superior suturability; reduced immunogenicity resulting in a reduce host-graft rejection response; ease of storage and shipment without the need for freezing or cryopreservation; minimal post-preparation requirement for handling and activation, *i.e*., rehydration, procedures prior to use; and the ability to be stored at room temperature for extended periods of time while maintaining structural and functional integrity. Table 1 summarizes the advantages of the biofabric of the invention as compared to the ones in the prior art.

In alternative disclosed herein is a collagen biofabric comprising a dehydrated, decelluarized, and substrate-free chorionic membrane, preferably a human chorionic membrane. It is expected that a collagen biofabric comprising a chorionic membrane will have comparable properties as the collagen biofabric of the invention Disclosed herein are comprising an amniotic membrane. Disclosed herein are all medically useful forms of the collagen biofabric comprising a chorionic membrane including but not limited to laminates, three-dimensional scaffolds, and hydrogel compositions.

**TABLE 1. BIOFABRICS OF THE INVENTION vs. TRADITIONAL AMNIOTIC MEMBRANE PREPARATIONS**

| **FEATURE** | **COLLAGEN BIOFABRIC OF THE INVENTION** | **TRADITIONAL AMNIOTIC MEMBRANES** |
|---|---|---|
| Seriological Testing | **COMPLETE:** antibody screen (ATY); alanine amino transferase screening (ALT); Hepatitis Core Antibody(nucleic acid and ELISA); Hepatitis B Surface Antigen(nucleic acid and ELISA); Hepatitis C Virus Antibody (nucleic acid and ELISA); HIV-1 and HIV-2; HTLV-1 and HTLV-2; Syphillis test (RPR); CMV antibody test; Hepatitis C and HIV test (nucleic acid test) | **INCOMPLETE:** No CMV test |
| Preservation | **DRY:** Dehydrated with low-heat vacuum; no need for freezer or refrigeration; shelf storage | **FROZEN:** stored in culture medium; requires dry ice shipping and freezer/refrigeration |
| Surgical Preparation | **MINUTES:** Prepares within minutes; hydrates directly on the surgical site; *e.g.* on the eye with saline drops; no thawing, soaks, or rinses needed | **LENGTHY:** requires extensive preparation/thawing/soak time; must remove filter backing |
| Surgical Handling | **SIMPLE:** can be trimmed while dry; and applied to the surgical site and then hydrated in the site | **TEDIOUS:** Must be removed from the filter paper; tends to 'ball up" during surgery; difficult to surgically manipulate |
| Cellularity | **DECELLULARIZED:** minimal to no epithelial cells; minimal to no cellular debris; faster remodeling | **WITH DEAD CELLS:** dead cells present; clinical evidence suggest longer healing time/higher rejection rate (anecdotal evidence) |
| Substrate | **NO:** Substrate-free (*e.g*., no filter backing) | **YES:** supported on nitrocellulose/filter paper |
| Sterilization (Optional) | **YES:** Electron beam irradiation; at least 18 kGy; increased assurance of tissue safety | **NO:** stored in media containing antibiotics and glycerol (toxic); no formal terminal sterilization |
| Tissue Clarity | **TRANSLUCENT:** Optically clear | **OPAQUE:** milky or cloudy appearance |

In a preferred embodiment, the collagen biofabric comprised in the invention is translucent, *i. e.,* optically clear. In another preferred embodiment, the collagen biofabric comprised in the invention is thin and lightweight. In a specific embodiment, the dehydrated collagen biofabric comprised in the invention is 0.3-0.6 mg/cm². In a specific embodiment, the collagen biofabric comprised in the invention is at least 30 microns in thickness. In another specific embodiment, the collagen biofabric comprised in the invention is approximately 10-40 microns in thickness.

Disclosed herein is the use of the collagen biofabric comprised in the invention in various configurations, e.g., inserts, shields. In general, the biofabric may be configured into any medically useful form. For example, the collagen biofabric may be configured for use in its entirety, *i.e.,* use of the collagen biofabric from an entire placenta. Alternatively, the collagen biofabric may be cut into strips, patches or rolls, or may be woven into threads. In another embodiment, the collagen biofabric, either as a single-thickness sheet or laminate, may be cut at regular intervals and expanded, e.g., to form a mesh.

In some embodiments, the collagen biofabric is flat, e.g., having a surface with no slope or curvature. In other embodiments the collagen biofabric comprised in the invention is not completely flat and has a dimpled surface, e.g., depression or indentation on the surface.

The invention encompasses a laminate comprising at least two layers of the collagen biofabric of the invention. The layers may be physically bound together, for example through the use of a glue, a fastener, or by heat-stamping a portion of the biofabrics, *e.g.,* the periphery of at least one of said layers, to fuse the layers. Because the biofabric has a "grain," the biofabric may be laminated in a variety of ways. In one embodiment, all layers of said laminate have the same grain orientation. In another embodiment, at least one of the layers is in a grain orientation that is rotated about 90 degrees (*i.e.,* is about perpendicular to) the grain orientation of at least one other layer. In a more specific embodiment, said perpendicular layers are adjacent to one another. In yet another embodiment, said laminate comprises at least three layers of the collagen biofabric, wherein the grain structures of each of said at least three layers is rotated about 60 degrees to the grain structure of two of the remaining layers. In another embodiment, said laminate contains at least a first material and a second material, wherein said first material is the collagen biofabric comprised in the invention, and said second material is any other substance that can form a laminate with the collagen biofabric. In a specific embodiment, said second material is a sheet or membrane derived from a membrane other than the amniotic membrane. In another specific embodiment, said second material is a non-natural material, for example, polylactone, polyacetate, plastic film, or the like. In some embodiments, the amniotic membrane laminate is multi-layer, comprising at least 6, at least 8, at least 10, at least 20, at least 80, at least 100, at least 1000 amniotic membranes that have been prepared in accordance with the methods of the invention. The amniotic membrane laminate of the invention may contain an unlimited number of layers.

The laminates have increased structural rigidity that allow the laminate to be shaped into complex three-dimensional structures. Such three-dimensional structures may include sheets, tubes, microspheres.

The collagen biofabric may also be associated with another material, either as a single sheet or as a laminate. For example, the biofabric may be associated with, *i.e*., bound to, flexible plastic film, gauze, plastic sheeting, stents, valves, orthopedic devices, bandages, patches, *etc.*

The collagen biofabric may comprise one or more compounds or substances that are not part of the collagen matrix of the biofabric. For example, the collagen biofabric may be impregnated, either during production or during preparation for surgery, with a biomolecule. Such biomolecules include but are not limited to, antibiotics (such as Clindamycin, Minocycline, Doxycycline, Gentamycin), hormones, growth factors, anti-tumor agents, anti-fungal agents, anti-viral agents, pain medications, anti-histamines, anti-inflammatory agents, anti-infectives including but not limited to silver (such as silver salts, including but not limited to silver nitrate and silver sulfadiazine), elemental silver, antibiotics, bactericidal enzymes (such as lysozome), wound healing agents (such as cytokines including but not limited to PDGF, TGF; thymosin), Hyaluronic acid as a wound healing agent, wound sealants (such as fibrin with or without thrombin), cellular attractant and scaffolding reagents (such as fibronectin) and the like. In a specific example, the collagen biofabric may be impregnated with at least one growth factor, for example, fibroblast growth factor, epithelial growth factor, *etc.* The biofabric may also be impregnated with small organic molecules such as specific inhibitors of particular biochemical processes *e.g.,* membrane receptor inhibitors, kinase inhibitors, growth inhibitors, anticancer drugs, antibiotics, *etc.*

In yet other embodiments, the collagen biofabric comprised in the invention may be combined with a hydrogel. Any hydrogel composition known to one skilled in the art is encompassed within the invention, e.g., any of the hydrogel compositions disclosed in the following reviews: Graham, 1998, Med. Device Technol. 9(1): 18-22; Peppas et al., 2000, Eur. J. Pharm. Biopharm. 50(1): 27-46; Nguyen et al., 2002, Biomaterials, 23(22): 4307-14; Henincl et al., 2002, Adv. Drug Deliv. Rev 54(1): 13-36; Skelhorne et al., 2002, Med. Device. Technol. 13(9): 19-23; Schmedlen et al., 2002, Biomaterials 23: 4325-32; . In a specific embodiment, the hydrogel composition is applied on the collagen biofabric, *i.e*., discharged on the surface of the collagen biofabric. The hydrogel composition for example, may be sprayed onto the collagen biofabric, saturuated on the surface of the biofabric, soaked with the collagen biofabric, bathed with the collagen biofabric or coated onto the surface of the collage biofabric.

The hydrogels useful in the methods and compositions of the invention can be made from any water-interactive, or water soluble polymer known in the art, including but not limited to, polyvinylalcohol (PVA), polyhydroxyehthyl methacrylate, polyethylene glycol, polyvinyl pyrrolidone, hyaluronic acid, dextran or derivatives and analogs thereof.

In some embodiments, the collagen biofabric comprised in the invention is further impregnated with one or more biomolecules prior to being combined with a hydrogel. In other embodiments, the hydrogel composition is further impregnated with one or more biomolecules prior to being combined with a collagen biofabric comprised in the invention. Such biomolecules include but are not limited to, antibiotics (such as Clindamycin, Minocycline, Doxycycline, Gentamycin), hormones, growth factors, anti-tumor agents, anti-fungal agents, anti-viral agents, pain medications, anti-histamines, anti-inflammatory agents, anti-infectives including but not limited to silver (such as silver salts, including but not limited to silver nitrate and silver sulfadiazine), elemental silver, antibiotics, bactericidal enzymes (such as lysozome), wound healing agents (such as cytokines including but not limited to PDGF, TGF; thymosin), Hyaluronic acid as a wound healing agent, wound sealants (such as fibrin with or without thrombin), cellular attractant and scaffolding reagents (such as fibronectin) and the like. In a specific example, the collagen biofabric or the hydrogel composition may be impregnated with at least one growth factor, for example, fibroblast growth factor, epithelial growth factor, *etc.* Preferably, the biomolecule is a therapeutic agent.

In some embodiments, the hydrogel composition is combined with a laminate comprising the biofabric comprised in the invention.

The hydrogel/collagen biofabric composition has utility in the medical field including but not limited to, treatment of wounds, bums, and skin conditions *(e.g.,* to treat scarring), cosmetic uses (*e.g*., cosmetic surgery), and any use as an implant. In some embodiments, the hydrogel/collagen biofabric composition is applied topically to a subject, *i.e.,* on the surface of the skin, for example, for the treatment of a wound. In other embodiments, the hydrogel/collagen biofabric composition may be used in the interior of a subject, for example as an implant, to become a permanent or semi-permanent structure in the body. In some embodiments, the hydrogel compositions in formulated to be non-biodegradable. In yet other embodiments, the hydrogel composition is formulated to be biodegradable. In a specific embodiment, the hydrogel composition is formulated to degrade within days. In another specific embodiment, the hydrogel composition is formulated to degrade within months.

In some embodiments, the collagen biofabric comprised in the invention is populated with cells, so that the cells are uniform and confluent. Cells that can be used to populate a biofabric comprised in the invention include but are not limited to, stem cells, preferably human stem cells, human differentiated adult cells, totipotent stem cells, pluripotent stem cells, multipotent stem cells, tissue specifc stem cells, embryonic like stem cells, committed progenitor cells, fibroblastoid cells. Disclosed herein is populating the biofabric of the invention with specific classes of progenitor cells including but not limited to chondrocytes, hepatocytes, hematopoietic cells, pancreatic parenchymal cells, neuroblasts, and muscle progenitor cells. The cells are not of human embryonic origin.

### 5.2 PROCESS FOR PRODUCING COLLAGEN BIOFABRICS

Disclosed herein is a method for preparing a collagen biofabric comprised in the invention. In particular, disclosed herein is a method for preparing a collagen biofabric comprising: providing a placenta, comprising an amniotic membrane and a chorionic membrane; separating the amniotic membrane from the chorionic membrane; and decellularizing the amniotic membrane. The method further entails washing and drying the decelluarized amniotic membrane.

Preferably, the placenta is from a human placenta for use in human subjects, or placenta from animal species for use in human subject, or placenta from animal species for veterinary use in animal subjects.

As disclosed herein the placenta for use in the methods of the invention is taken as soon as possible after delivery of the newborn. In yet another preferred embodiment, the placenta is taken immediately following the cesarean section delivery of a normal healthy infant. Provided the placenta is collected under asceptic conditions. In some embodiments, the placenta is stored for 48 hours from the time of delivery prior to any further treatment. In other embodiments, the placenta is stored for up to 5 days from the time of delivery prior to any further treatment.

Preferably, the placenta, umbilical cord, and umbilical cord blood are transported from the delivery or birthing room to another location, *e.g*., a laboratory, for further processing. The placenta is preferably transported in a sterile, transport device such as a sterile bag or a container, which is optionally thermally insulated. In some embodiments, the placenta is stored at room temperature until further treatment. In other embodiments, the placenta is refrigerated until further treatment, *i.e.,* stored at a temperature of about 2° to 8°C. In yet other embodiments, the placenta is stored under sterile conditions for up to 5 days before further treatment. In a most preferred embodiment, the placenta is handled and processed under aseptic conditions, as known to one skilled in the art. The laboratory is preferably equipped with an HEPA filtration system (as defined by clean room classification, having a class 1000 or better). In a preferred embodiment, the HEPA filtration system is turned on at least 1 hour prior to using the laboratory room for carrying out the methods of the invention.

In certain embodiments, the placenta is exsanguinated, *i.e.,* completely drained of the cord blood remaining after birth. In some embodiments, the placenta is 70% exsanguinated, 80% exsanguinated, 90% exsanguinated, 95% exsanguinated, 99% exsanguinated.

Disclosed herein is screening the expectant mother prior to the time of birth, using standard techniques known to one skilled in the art, for communicable diseases including but not limited to, HIV, HBV, HCV, HTLV, syphilis, CMV, and other viral pathogens known to contaminate placental tissue. Preferably, the methods used to screen for a communicable disease follow the regulations as set forth by the Federal Drug Administration. The expectant mother may be screened *(e.g.,* a blood sample is taken for diagnostic purposes) within one month of birth, preferably within two weeks of birth, even more preferably within one week of birth, and, most preferably, at the time of birth. Only tissues collected from donors whose mothers tested negative or non-reactive to the above-mentioned pathogens are used to produce a biofabric of the invention. Preferably, a thorough paternal and medical and social history of the donor of the placental membrane is obtained, including for example, a detailed family history.

The donor is screened using standard serological and bacteriological tests known to one skilled in the art. Any assay or diagnostic test that identifies the pathogen(s) is within the scope of the method of the invention, but preferable assays are ones that combine high accuracy with capacity for high throughput. Disclosed herein is screening the donor using standard techniques known to one skilled in the art for antigens and/or antibodies. A non-limiting example of antigens and antibodies include: antibody screen (ATY); alanine amino transferase screening (ALT); Hepatitis Core Antibody(nucleic acid and ELISA); Hepatitis B Surface Antigen; Hepatitis C Virus Antibody; HIV-1 and HIV-2; HTLV-1 and HTLV-2; Syphillis test (RPR); CMV antibody test; and Hepatitis C and HIV test. The assays used may be nucleic acid based assays or ELISA based assays as known to one skilled in the art.

Disclosed herein is further testing the blood from the umbilical cord of the newborn using standard techniques known to one skilled in the art (See, *e.g.,* Cotorruelo et al., 2002, Clin Lab. 48(5-6):271-81; Maine et al., 2001, Expert Rev. Mol. Diagn., 1(1):19-29; Nielsen et al., 1987, J. Clin. Microbiol. 25(8):1406-10; all of which are incorporated herein by reference in their entirety). In one embodiment, the blood from the umbilical cord of the newborn is tested for bacterial pathogens (including but not limited to gram positive and gram negative bacteria) and fungi using standard techniques known to one skilled in the art. In a specific embodiment, the blood type and Rh factor of the blood of the umbilical cord of the newborn is determined using standard techniques known to those skilled in the art. In another embodiment, CBC with differential is obtained from the blood from the umbilical cord of the newborn using standard methods known to one skilled in the art. In yet another embodiment, an aerobic bacterial culture is taken from the blood from the umbilical cord of the newborn, using standard methods known to one skilled in the art. Only tissues collected from donors that have a CBC within a normal limit (*e.g.*, no gross abnormality or deviation from the normal level), test negative for serology and bacteriology, and test negative or non-reactive for infectious disease and contamination are used to produce a biofabric of the invention.

One exemplary method for preparing a collagen biofabric comprised in the invention comprises the following steps.

**Step I.** The invention encompasses processing the placental membrane so that the umbilical cord is separated from the placental disc (optionally), and separation of the amniotic membrane from the chorionic membrane. In a preferred embodiment, the amniotic membrane is separated from the chorionic membrane prior to cutting the placental membrane. The separation of the amniotic membrane from the chorionic membrane is preferably done starting from the edge of the placental membrane. In another preferred embodiment, the amniotic membrane is separated from the chorionic membrane using blunt dissection, *e.g.,* with gloved fingers. Following separation of the amniotic membrane from the chorionic membrane and placental disc, the umbilical cord stump is cut *e.g.,* with scissors, and detached from the placental disc. In certain embodiments, when separation of the amniotic and chorionic membranes is not possible without tearing the tissue, the invention encompasses cutting the amniotic and chorionic membranes from the placental disc as one piece and then peeling them apart.

The amniotic membrane is then preferably stored in a sterile saline solution. In some embodiments, the sterile saline solution is buffered. In a specific preferred embodiment, the sterile saline solution for storing the amniotic membrane is a 0.9% sterile NaCl solution. Preferably, the amniotic membrane is stored by refrigeration, at a temperature of at least 4°C. In certain embodiments, the amniotic membrane is refrigerated at a temperature of at least 2°C, at least 6°C, or up to 8°C. At this point, the amniotic membrane may be stored for up to 5 days, provided it is refrigerated and kept covered with sterile saline. Preferably, the separated amniotic membrane is refrigerated for a maximum of 72 hours from the time of delivery prior to the next step in the process.

**Step II.** Once the amniotic membrane is separated from the chorionic membrane, the invention encompasses decellularizing the amniotic membrane. Any decellularizing process known to one skilled in the art is encompassed by the methods of the invention, with the provision that the process for decellularizing the amniotic membrane of the invention does not include any freezing of the amniotic membrane. As used herein, decellularizing refers to removing all cellular material and cellular debris (*e.g.*, all visible cellular material and cellular debris) from the amniotic membranes of the invention. The decelluarization of the amniotic membrane ensures that substantially all of the cells normally associated with the collagen matrix of the amniotic membrane are removed. Decellularization of the amniotic membrane of the invention that removes "substantially all" of the cells associated with the collagen matrix preferably removes at least 90% of the cells, more preferably removes at least 95% of the cells, and most preferably removes at least 99% of the cells. The amniotic membranes decellularized in accordance with the methods of the invention are uniformly thin, *i.e*., 10-40 microns in thickness, smooth (as determined by touch) and clear in appearance.

In a preferred embodiment, decellularization of the amniotic membrane of the invention comprises removing substantially all cellular material and cellular debris from the maternal side of the amniotic membrane followed by removing all cellular material and cellular debris from the fetal side of the amniotic membrane. In a specific embodiment, decellularization of the amniotic membrane of the invention comprises physical scraping in combination with rinsing with a sterile solution. In another specific embodiment, physical scraping of the amniotic membrane comprises scraping with a sterile cell scraper. In yet another specific embodiment, the sterile solution for rinsing the amniotic membrane during decellularization is an aqueous solution, a solution comprising a physiological buffer or a saline solution such as for example a 0.9% NaCl solution.

The decellularization of the amniotic membrane comprises removing native cells and other antigens and cellular debris from the amniotic membrane, and, optionally, treating to inhibit generation of new immunological sites. In decellularizing the amniotic membrane, native viable cells as well as other cellular and acellular structures or components which may elicit an adverse immune response are removed. The decellularization technique employed in accordance with the invention should not result in gross disruption of the anatomy of the amniotic membrane or alter the biomechanical properties of its structural composition, *i.e.,* the structural and biochemical integrity of collagen, elastin, and possibly fibronectin are not affected by the decellularization. Specifically, harsh chemical treatment and protease treatment of the amniotic membrane are not within the scope of the decelluarization technique of the present invention.

Preferably, the decellularization of the amniotic membrane comprises use of a detergent-containing solution. Detergents that can be used in accordance with the methods of the present invention include, but are not limited to, nonionic detergents, Triton X-100, anionic detergents, sodium dodecyl sulfate. Detergents can be used alone or in combination in the methods of the present invention. Any mild anionic detergent, *i.e.*, a non-caustic detergent, with a pH of 6 to 8, and low foaming, can be used in accordance with the methods of the invention. In a specific embodiment, 0.01-1% deoxycholic acid sodium salt monohydrate is used in the decellularization of the amniotic membrane. Although not intending to be bound by a particular mode of action, decellularization of the amniotic membrane in accordance with the methods of the invention may disrupt cell membranes and aid in the removal of cellular debris from the amniotic membrane. However, steps should be taken to eliminate any residual detergent levels in the amniotic membrane, so as to for example, avoid interference with the later repopulating of the amniotic membrane with viable cells.

It is essential to limit the protease activity in preparation of the biofabric of the invention. Additives such as metal ion chelators, for example 1,10-phenanthroline and ethylenediaminetetraacetic acid (EDTA), create an environment unfavorable to many proteolytic enzymes. Providing sub-optimal conditions for proteases such as collagenase, may assist in protecting the amniotic membrane compositions such as collagen from degradation during the lysis step. Suboptimal conditions for proteases may be achieved by formulating the hypotonic lysis solution to eliminate or limit the amount of calcium and zinc ions available in solution. Many proteases are active in the presence of calcium and zinc ions and lose much of their activity in calcium and zinc ion free environments. Preferably, the hypotonic lysis solution will be prepared selecting conditions of pH, reduced availability of calcium and zinc ions, presence of metal ion chelators and the use of proteolytic inhibitors specific for collagenase such that the solution will optimally lyse the native cells while protecting the underlying amniotic membrane from adverse proteolytic degradation. For example a hypotonic lysis solution may include a buffered solution of water, pH 5.5 to 8, preferably pH 7 to 8, free from calcium and zinc ions and including a metal ion chelator such as EDTA. Additionally, control of the temperature and time parameters during the treatment of the amniotic membrane with the hypotonic lysis solution, may also be employed to limit the activity of proteases.

It is preferred that the decellularization treatment of the amniotic membrane also limits the generation of new immunological sites. Since enzymatic degradation of collagen is believed to lead to heightened immunogenicity, the invention encompasses treatment of the amniotic membrane with enzymes, *e.g.,* nucleases, that are effective in inhibiting cellular metabolism, protein production and cell division, that minimize proteolysis of the compositions of the amniotic membrane thus preserving the underlying architecture of the amniotic membrane. Examples of nucleases that can be used in accordance with the methods of the invention are those effective in digestion of native cell DNA and RNA including both exonucleases and endonucleases. A non-limiting example of nucleases that can be used in accordance with the methods of the invention include exonucleases that inhibit cellular activity, *e.g*., DNAase I (SIGMA Chemical Company, St. Louis, Mo.) and RNAase A (SIGMA Chemical Company, St. Louis, Mo.) and endonucleases that inhibit cellular activity, *e.g.,* EcoR I (SIGMA Chemical Company, St. Louis, Mo.) and Hind Ill (SIGMA Chemical Company, St. Louis, Mo.). It is preferable that the selected nucleases are applied in a physiological buffer solution which contains ions, *e.g.,* magnesium, calcium, which are optimal for the activity of the nuclease. Preferably, the ionic concentration of the buffered solution, the treatment temperature and the length of treatment are selected by one skilled in the art by routine experimentation to assure the desired level of nuclease activity. The buffer is preferably hypotonic to promote access of the nucleases to cell interiors.

In a specific embodiment, decellularizing the amniotic membrane of the invention comprises the following steps. First, the amniotic membrane is transferred into a clean sterile container, and optionally rinsed with sterile water and dried with sterile gauze. The amnion is then placed on a sterile tray with the maternal side facing upward. Using a sterile cell scraper (*e.g*., 32 cm, PE blade, PS handle, NalgeNunc International), the amnion is partially decellularized by physically removing all visible cellular material from the maternal side of the amniotic membrane. Sterile water is used to assist in the removal of cells and cellular debris, if needed. After completing the partial decellularization on the maternal side of the amniotic membrane, the amniotic membrane is turned over so that the fetal side faces up. All visible cellular debris on the fetal side is gently removed with a cell scraper using minimal pressure on the amniotic membrane to prevent tearing. Sterile water may be used to assist in the removal of the cells and debris.

In one embodiment, the decellularized amniotic membrane is placed in a sterile container filled with a sterile physiological solution, e.g., sterile 0.9% NaCl solution, before further processing. In accordance with the methods of the invention the next processing step of the amniotic membrane should start no later than 2-3 hours after the amniotic membrane has been placed into the sterile physiological solution. In a specific embodiment, where Step III immediately follows Step II, it is not necessary to place the amniotic membrane into a container with sterile solution.

The amniotic membrane may be cut during the cleaning process, using *e.g*. a sterile scalpel, to shape it for easier cleaning or to remove the areas that cannot be cleaned.

**Step III.** Following decellularization, the amniotic membrane is washed to assure removal of cellular debris which may include cellular proteins, cellular lipids, and cellular nucleic acids, as well as any extracellular debris such as extracellular soluble proteins, lipids and proteoglycans. Although not intending to be bound by any mechanism of action, removal of this cellular and extracellular debris reduces the immunogenicity of the amniotic membrane.

Once the amniotic membranes of the invention are decellularized, the membranes are further washed in order to effectively achieve the complete removal of all visible cellular material and cellular debris from both sides of the amniotic membrane. The solution is preferably an aqueous hypotonic or low ionic strength solution formulated to effectively lyse the native tissue cells. Such an aqueous hypotonic solution may be de-ionized water or an aqueous hypotonic buffer. Preferably the aqueous hypotonic buffer additionally contains additives that provide sub-optimal conditions for the activity of proteases, for example collagenase, which may be released as a result of cellular lysis.

Preferably, the amniotic membrane is gently agitated in the detergent, *e.g*., on a rocking platform, to assist in the decellularization. In certain embodiments, the amniotic membrane is agitated for at least 15 minutes, at least 20 minutes, at least 30 minutes, or up to 120 minutes. The amniotic membrane may, after detergent decellularization, again be physically decellularized as described *supra*; the physical and detergent decellularization steps may be repeated as necessary, as long as the integrity of the amniotic membrane is maintained, until no visible cellular material and cellular debris remain.

In a specific embodiment, the washing of the amniotic membrane comprises the following steps: the decellularized amniotic membrane is placed into a sterile container which is then filled with a decellularizing solution in an amount sufficient to cover the amniotic membrane; the container with the amniotic membrane and the decellularizing solution is then placed on a rocking platform (*e.g*., Model 100, VWR Scientific Products Corp., P.O. Box 640169, Pittsburgh, PA 15264-0169). The amniotic membrane in the decellularizing solution is then agitated for between 15 minutes and 120 minutes on the rocking platform. After the agitation step, the amniotic membrane is removed from the container and placed in a clean sterile tray filled with a sterile solution, e.g., 0.9% NaCl solution. Using a new sterile Cell Scraper, residual decellularizing solution is removed and any remaining cellular material is removed form both sides of the amniotic membrane. This step may be repeated as many times as necessary to remove all visible residual cellular material from both sides of the amniotic membrane.

In certain embodiments, the amniotic membrane is dried immediately *(i.e.,* within 30 minutes) after the decellularization step. Alternatively, when further processing is not done immediately, the amniotic membrane may be refrigerated, e.g., stored at a temperature of 2-8°C, for up to 28 days prior to drying. When the decellularized amniotic membrane is stored for more than three days but less than 28 days, the sterile solution covering the amniotic membrane is preferably changed periodically, e.g., every 1-3 days.

In certain embodiments, when the amniotic membrane is not refrigerated after washing, the amniotic membrane is washed at least 3 times prior to proceeding to Step IV of the preparation. In other embodiments, when the amniotic membrane has been refrigerated and the sterile solution has been changed once, the amniotic membrane is washed at least twice prior to proceeding to Step IV of the preparation. In yet other embodiments, when the amniotic membrane has been refrigerated and the sterile solution has been changed twice or more, the amniotic membrane is washed at least once prior to proceeding to Step IV of the preparation.

In specific embodiments, the decellularized amniotic membrane is stored under sterile conditions, and no further processing is performed, *i.e.,* no drying. Prior to proceeding to Step IV, it is essential that all bacteriological and seriological testing be assessed to ensure that all tests were negative.

Step IV. The final step of the method of the invention comprises drying the decellularized amniotic membrane of the invention to produce the collagen biofabric. Any method of drying the amniotic membrane so as to produce a flat, dry sheet of collagen may be used. Preferably, however, the amniotic membrane is dried under vacuum.

In a specific embodiment, an exemplary method for drying the decellularized amniotic membrane of the invention comprises the following steps:

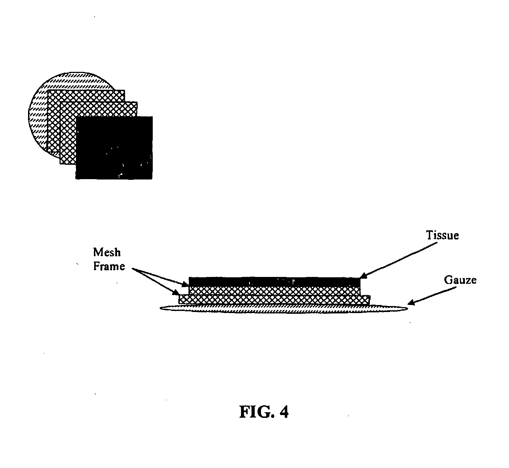

*Assembly of the* decellularized *amniotic membrane for drying.* The decellularized amniotic membrane is removed from the sterile solution, and the excess fluid is gently squeezed out. The decellularized amniotic membrane is then gently stretched until it is flat with the fetal side faced in a downward position, e.g., on a tray. The decellularized amniotic membrane is then flipped over so that fetal side is facing upwards, and placed on a drying frame, preferably a plastic mesh drying frame (e.g., Quick Count® Plastic Canvas, Uniek, Inc., Waunakee, WI). In other embodiments, the drying frame may be an autoclavable stainless steel mesh. In a most preferred embodiment, about 0.5 centimeter of the amniotic membrane overlaps the edges of the drying frame. In certain embodiments, the overlapping amniotic membrane extending beyond the drying frame is wrapped over the top of the frame, *e.g*., using a clamp or a hemostat. Once the amniotic membrane is positioned on the drying frame, a sterile gauze is placed on the drying platform of a heat dryer (or geldryer) (*e.g*., Model 583, Bio-Rad Laboratories, 200 Alfred Nobel Drive, Hercules, CA 94547), so that an area slightly larger than the amniotic membrane resting on the plastic mesh drying frame is covered. Preferably, the total thickness of the gauze layer does not exceed the thickness of one folded 4x4 gauze. Any heat drying apparatus may be used that is suitable for drying sheet like material. The drying frame is placed on top of the gauze on the drying platform so that the edges of the plastic frame extend above beyond the gauze edges, preferably between 0.1 - 1.0 cm, more preferably 0.5-1.0 cm. In a most preferred embodiment, the drying frame having the amniotic membrane is placed on top of the sterile gauze with the fetal side of the amniotic membrane facing upward. In some embodiments, another plastic framing mesh is placed on top of the amniotic membrane. A view of the mesh frame and the membrane dried therein is shown in FIG. 4. In another embodiments, a sheet of thin plastic (e.g., SW 182, clear PVC, AEP Industries Inc., South Hackensack, NJ 07606) or a biocompatible silicone is placed on top of the membrane covered mesh so that the sheet extends well beyond all of the edges. In this embodiment, the second mesh frame is not needed.

In an alternative embodiment, the amniotic membrane is placed one or more sterile sheets of Tyvek material (*e.g*., a sheet of Tyvek for medical packaging, Dupont Tyvek®, P.O. Box 80705, Wilmington, DE 19880-0705), optionally, with one sheet of Tyvek on top of the membrane (prior to placing the plastic film). This alternate process will produce a smoother version of the biofabric *(i.e.,* without the pattern of differential fiber compression regions along and perpendicular to the axis of the material), which may be advantageous for certain applications, such as for example for use as a matrix for expansion of cells, as described herein.

*Drying the amniotic membrane.* In a preferred embodiment, the invention encompasses heat drying the amniotic membrane of the invention under vacuum. While the drying under vacuum may be accomplished at any temperature from about 0°C to about 60°C, the amniotic membrane is preferably dried at between about 35°C and about 50°C, and most preferably at about 50°C. It should be noted that some degradation of the collagen is to be expected at temperatures above 50°C. The drying temperature is preferably set and verified using a calibrated digital thermometer using an extended probe. Preferably, the vacuum pressure is set to about -22 inches of Hg. The drying step is continued until the collagen matrix of the amniotic membrane contains less than 3-12% water as determined for example by a moisture analyzer. To accomplish this, the amniotic membrane may be heat-vacuum dried, e.g., for approximately 60 minutes to achieve a dehydrated amniotic membrane. In some embodiments, the amniotic membrane is dried for about 30 minutes to 2 hours, preferably about 60 minutes. Although not intending to be bound by any mechanism of action, it is believed that the low heat setting coupled with vacuum pressure allows the amniotic membrane to achieve the dehydrated state without denaturing the collagen.

After completion of the drying process in accordance with the invention, the amniotic membrane is cooled down for approximately two minutes with the vacuum pump running.

*Packaging and Storing of the Amniotic Membrane.* Once the amniotic membrane is dried in accordance with the methods of the invention as described, the membrane is gently lifted off the drying frame. "Lifting off' the membrane may comprise the following steps: while the pump is still running, the plastic film is gently removed from the amniotic membrane starting at the corner, while holding the amniotic membrane down; the frame with the amniotic membrane is lifted off the drying platform and placed on a cutting board with the amniotic membrane side facing upward; an incision is made, cutting along the edge 1-2 mm away from the edge of the frame; the amniotic membrane is then peeled off the frame; and cut in to appropriate sizes as determined by its subsequent use. Preferably, handling of the amniotic membrane at this stage is done with sterile gloves.

The amniotic membrane is placed in a sterile container, e.g., peel pouch, and is sealed. The biofabric produced in accordance with the methods of the invention may be stored at room temperature for an extended period of time as described *supra.*

Disclosed herein is also a method of preparing a collagen biofabric comprising a chorionic membrane. It is expected that the methods described above would be applicable to the method of preparing a biofabric comprising a chorionic membrane. Disclosed herein is also a method for preparing a collagen biofabric comprising: providing a placenta, comprising an amniotic membrane and a chorionic membrane; separating the amniotic membrane from the chorionic membrane; and decellularizing the chorionic membrane. The method further entails washing and drying the decelluarized chorionic membrane.

### 5.2.1 METHODS OF PREPARING THREE-DIMENSIONAL SCAFFOLDS AND LAMINATES

The invention provides methods of preparing three-dimensional scaffolds, three-dimensional configurations and laminates comprising the collagen biofabric comprised in the invention.

In some embodiments, the invention provides a method of preparing an amniotic membrane laminate comprising: providing a placenta, preferably a human placenta, comprising an amniotic membrane and a chorionic membrane, separating the amniotic membrane from the chorionic membrane, using methods disclosed herein; decellularizing the amniotic membrane, using methods disclosed herein; washing the decellularized amniotic membrane at least once using methods disclosed herein; layering at least two of the decellularized amniotic membranes in contact with each other so that an amniotic membrane laminate is formed; and drying the amniotic membrane laminate, using methods disclosed herein.

Alternatively, in another embodiment, the method for preparing an amniotic membrane laminate comprises, drying at least two amniotic membranes prepared in accordance with the methods of the invention, and layering the at least two amniotic membranes in contact with each other so that an amniotic membrane laminate is formed.

In some embodiments, the amniotic membrane layers produced in accordance with the methods of the invention may be placed in contact with each other in the presence of an adhesive to form an amniotic membrane laminate. The adhesive used in accordance with the methods and compositions of the invention may be any biological glue known to one skilled in the art, preferably a biocompatible glue, including but not limited to, natural glue, e.g., fibronectin, fibrin, synthetic glue. In other embodiments, the amniotic membrane layers prepared in accordance to the methods of the invention are cross-linked to each other to form an amniotic membrane laminate. Any cross-linking reagent and method known to one skilled in the art is within the scope of the present invention, including but not limited to, chemical cross-linking, peptide cross-linking, UV cross-linking, radiation cross-linking, fibronectin cross-linking, fibrinogen cross-linking, hydrogel cross-linking. In other embodiments, the amniotic membrane laminates produced in accordance with the methods of the invention do not comprise an adhesive.

### 5.3 STORAGE AND HANDLING OF THE COLLAGEN BIOFABRIC

Disclosed herein is storing the collagen biofabric comprised in the invention as dehydrated sheets at room temperature (e.g., 25°C). The collagen biofabric comprised in the invention can be stored at a temperature of at least 10°C, at least 15°C, at least 20°C, at least 25°C, or at least 29°C. Preferably, the collagen biofabric comprised in the invention is not refrigerated. As disclosed herein the collagen biofabric comprised in the invention may be refrigerated at a temperature of about 2 to 8°C. As disclosed herein, the collagen biofabric comprised in the invention can be stored at any of the above-identified temperatures for an extended period of time. As disclosed herein the biofabric comprised in the invention is stored under sterile and non-oxidizing conditions. The biofabric produced according to the methods of the invention can be stored at any of the specified temperatures for 12 months or more with no alteration in biochemical or structural integrity (*e.g.*, no degradation), without any alteration of the biochemical or biophysical properties of the collagen biofabric. The biofabric produced according to the methods of the invention can be stored for several years with no alteration in biochemical or structural integrity (*e.g.*, no degradation), without any alteration of the biochemical or biophysical properties of the collagen biofabric. It is expected that the biofabric of the invention prepared in accordance with the methods of the invention will last indefinitely. The biofabric may be stored in any container suitable for long-term storage. Preferably, the collagen biofabric of the invention is stored in a sterile double peel-pouch package.

The invention encompasses handling of the collagen biofabric in its dry state. In a specific embodiment, the collagen biofabric is trimmed prior to use, for example prior to use as a surgical graft. The invention encompasses any dimensionality of the biofabric of the invention that is compatible for its use, as determined by one skilled in the art. In some embodiments, the invention encompasses a collagen biofabric which is 1x2cm; 2x3 cm; 4x4 cm, 5x5, or 6x8. The biofabric of the invention can be cut into any size needed which is within the limitation of the size of the amniotic membrane.

The surface orientation of the collagen biofabric of the invention can be visually identified. The collagen biofabric of the invention has a "grid" pattern, which allows for the visual identification of the maternal and detal surfaces by one skilled in the art. In a specific embodiment, the surface orientation of the collagen biofabric is identified under magnification. It will be appreciated by one skilled in the art that the fetal side of the collagen biofabric can be identified by its concave, *i.e.,* recessed, grid pattern. Conversely, the maternal side can be identified by its convex, *i.e.,* elevated grid pattern.

The collagen biofabric comprised in the invention requires minimal preparation time prior to use. In a preferred embodiment, the collagen biofabric comprised in the invention is ready to use within 5 minutes or less, within 10 minutes or less, within 15 minutes or less. The preparation time of the collagen biofabric comprised in the invention prior to use for example, as a surgical graft, comprises activation by re-hydration of the dehydrated collagen biofabric. In some cases, the collagen biofabric comprised in the invention is hydrated while on the surgical site. In other cases, the collagen biofabric comprised in the invention is hydrated under sterile conditions in a dish. Disclosed herein is hydration of the collagen biofabric comprised in the invention using a sterile physiological buffer. Disclosed herein is hydrating the collagen biofabric comprised in the invention with a sterile saline solution, e.g., sterile 0.9% NaCl solution. In some cases the sterile saline solution is buffered. The hydration of the collagen biofabric comprised in the invention requires at least 2 minutes, at least 5 minutes, at least 10 minutes, at least 15 minutes, or at least 20 minutes. As disclosed herein, the hydration of the collagen biofabric comprised in the invention is complete within 5 minutes. In yet another case, the hydration of the collagen biofabric comprised in the invention is complete within 10 minutes. In yet another case, the hydration of the collagen biofabric comprised in the invention takes no more than 10 minutes.

The collagen biofabric comprised in the invention once hydrated for use, for example as a surgical graft, has enhanced suturability relative to the amniotic membranes in the art, as determined by one skilled in the art. The collagen biofabric comprised in the invention does not tear as easily nor is it as friable as the amniotic membranes in the art. The invention encompasses collagen biofabrics that can be sutured effectively.

### 5.3.1 STERILIZATION

Sterilization of the biofabric of the invention is preferably done by electron beam irradiation using methods known to one skilled in the art, e.g., Gorham, D. Byrom (ed.), 1991, Biomaterials, Stockton Press, New York, 55-122. Any dose of radiation sufficient to kill at least 99.9% of bacteria or other potentially contaminating organisms is disclosed herein. As disclosed herein, a dose of at least 18-25 kGy is used to achieve the terminal sterilization of the biofabric of the invention. Sterilization of the biofabric of the invention does not include, however, storage in antibiotics or glycerol.

### 5.4 METHODS OF USING THE COLLAGEN BIOFABRIC OF THE INVENTION

The collagen biofabric comprised in the invention has numerous utility in the medical and surgical field due, in part, to its physical properties, such as biomechanical strength, flexibility, suturability, low immunogenicity in comparison to the traditional membranes used in the art. For example, the collagen biofabric comprised in the invention is expected to have an enhanced therapeutic utility for guided tissue regeneration over membranes of the prior art, e.g., synthetic non-resorbable PTFE membranes such as Goretex™; synthetic resorbable membranes formed from glycolide and lactide copolymers; membranes disclosed in WO-88/08305; DE-2631909, U.S. Patent No. 5,837,278.

The method of preparing the collagen biofabric comprised in the invention ensures the preservation of the tertiary and quaternary structure of the biofabric thus making the biofabric ideal for its intended use in the medical and surgical field. As described in detail below, the invention provides collagen biofabrics whose physical properties allow it to be suitable for uses in a variety of medical and dental applications including but not limited to blood vessel repair, uterus repair, tendon replacements, cornea replacements, artificial skin, treatment of periodontal disease, and wound healing. Depending on its intended use, the invention encompasses use of the collagen biofabric as a two dimensional membrane, e.g., membranes that can be shaped to form tubular vessels; as a three-dimensional scaffold, e.g., an implant, or as a one-dimensional fiber.

### 5.4.1 METHODS FOR TREATMENT USING THE COLLAGEN BIOFABRIC OF THE INVENTION

### 5.4.1.1 METHODS FOR TREATMENT OF SKIN CONDITIONS

Human skin is a composite material of the epidermis and the dermis. The upper part of the epidermis is the stratum corneum; which is the stiffest layer of the skin, as well as the one most affected by the surrounding environment. Below the stratum corneum is the internal portion of the epidermis. Below the epidermis is the papillary dermis, which is made of relatively loose connective tissues that define the micro-relief of the skin. The reticular dermis, disposed beneath the papillary dermis, is tight, connective tissue that is spatially organized. The reticular dermis is also associated with coarse wrinkles. Underneath the dermis lies the subcutaneous layer.

The principal functions of the skin include protection, excretion, secretion, absorption, thermoregulation, pigmentogenesis, accumulation, sensory perception, and regulation of immunological processes. These functions are detrimentally affected by the structural changes in the skin due to aging and excessive sun exposure. The physiological changes associated with skin aging include impairment of the barrier function and decreased turnover of epidermal cells, for example, *See,* Cerimele, D. et al., 1990, Br. J. Dermatol., 122 Suppl. 35, p. 13-20. The methods and compositions in the prior art, however, have had limited success in improving skin conditions, e.g., improving skin elasticity and softness, or removing wrinkles.

The collagen biofabric comprised in the invention has medical as well as a cosmetic applications. The collagen biofabric comprised in the invention has clinical and therapeutic utility in treating skin conditions including but not limited to skin lesions, aged skin, wrinkles, fine lines, thinning, reduced skin elasticity, rough skin, congenital and degenerative skin conditions, collagen VII deficiency, and sun damaged skin. In certain embodiments, the collagen biofabric comprised in the invention can be used as a subcutaneous implant for skin conditions such as acne scars, glabellar furros, excision scars, or any other soft tissue defect known in the art. The collagen biofabric comprised in the invention has clinical and therapeutic utility in treating changes associated with skin aging. In certain embodiments, the collagen biofabric comprised in the invention has utility in improving skin wrinkles, and/or other conditions such as skin elasticity and softness. The collagen biofabric comprised in the invention may be used as an implant, as a laminate, or as a three dimensional rolled up form for the treatment of skin conditions. The collagen biofabric comprised in the invention is expected to have an enhanced clinical utility relative to methods known in the art for treating such skin conditions, e.g., U.S. Patent Nos. 5,972,999; 5,418,875; 5,332,579, 5,198,465, in part, due to its stability it may provide a longer lasting effect.

In a preferred embodiment, the collagen biofabric comprised in the invention is further supplemented with one or more agents known in the art for treating a skin condition. Examples of such agents, include but are not limited to, vitamins, minerals, catechin-based preparations, N-acetlyglucosamine, and glucosamine *(See, e.g*., Neldner, 1993, Amer. Acad. Derm. Annl. Mtg. Wash. DC.; Lubell 1996, Cosmetic Dermatol, 9(7): 58-60; Swaine et al., 1995, J. Am. Board of Family Practice, 8(3): 206-16; Shan et al., 1994, Kidney International, 46(2): 388-95).

The collagen biofabric comprised in the invention may be impregnated with any biomolecule with utility in the treatment of a skin condition, including but not limited to, antibiotics (such as Clindamycin, Minocycline, Doxycycline, Gentamycin), hormones, growth factors, anti-tumor agents, anti-fungal agents, anti-viral agents, pain medications, anti-histamines, anti-inflammatory agents, anti-infectives including but not limited to silver (such as silver salts, including but not limited to silver nitrate and silver sulfadiazine), elemental silver, antibiotics, bactericidal enzymes (such as lysozome), wound healing agents (such as cytokines including but not limited to PDGF, TGF; thymosin), Hyaluronic acid as a wound healing agent, wound sealants (such as fibrin with or without thrombin), cellular attractant and scaffolding reagents (such as fibronectin) and the like. In a specific example, the collagen biofabric may be impregnated with at least one growth factor, for example, fibroblast growth factor, epithelial growth factor, *etc.* The biofabric may also be impregnated with small molecules such as small organic molecules such as specific inhibitors of particular biochemical processes e.g., membrane receptor inhibitors, kinase inhibitors, growth inhibitors, anticancer drugs, antibiotics, *etc.*

### 5.4.1.2 WOUNDS AND BURNS

The collagen biofabric comprised in the invention is expected to have an enhanced clinical utility as a wound dressing, for augmenting hard and/or soft tissue repair, as compared to other biomaterials known in the art, *e.g*., those described in U.S. Patent Nos. 3,157,524; 4,320,201; 3,800,792; 4,837,285; 5,116,620, due in part to its physical properties. The collagen biofabric comprised in the invention because it retains collagen's native quaternary structure provides improved tissue in-growth through cell migration into the interstices of the comprised in collagen matrix. The biofabric comprised in the invention allows cells to attach and grow into the collagen matrix, and to synthesize their own macromolecules. The cells thereby produce a new matrix which allows for the growth of new tissue. Such cell development is not observed on other known forms of collagen such as fibers, fleeces and soluble collagen.

Disclosed herein is treating a wound by placing the collagen biofabric comprised in the invention directly over the skin of the subject, *i.e.,* on the stratum corneum, on the site of the wound, so that the wound is covered, for example, using an Disclosed herein is adhesive tape. Disclosure herein is treating a wound using the collagen biofabric comprised in the invention as an implant, e.g., as a subcutaneous implant.

Enhancing the rate of wound healing by the addition of a macromolecule capable of promoting tissue ingrowth to the collagen biofabric of the invention. Such macromolecules include but are not limited to hyaluronic acid, fibronectin, laminin, and proteoglycans (*See, e.g*., Doillon et al. (1987) Biomaterials 8:195-200; and Doillon and Silver (1986) Biomaterials 7:3-8).

Disclosure herein is incorporating pharmacologically active agents including but not limited to platelet-derived growth factor, insulin-like growth factor, epidermal growth factor, transforming growth factor beta, angiogenesis factor, antibiotics, antifungal agents, spermicidal agents, hormones, enzymes, enzyme inhibitors in the collagen biofabric of the invention as described herein in section 5.4.2.7 for delivery to the skin, and any biomolecule described above. Preferably the pharmacologically active agents are provided in a physiologically effective amount.

In some embodiments, the collagen biofabric is further populated by living cells, including but not limited to allogenic stem cells, stem cells, and autologous adult cells, to prior to being applied to the site of the wound. The cells are not of human embryonic origin.

The collagen biofabric comprised in the invention is particularly useful for the treatment of wound infections, *e.g*., wound infections followed by a breakdown of surgical or traumatic wounds. In a particular embodiment, the collagen biofabric is impreganted with a therapeutically effective amount of an agent useful in the treatment of a wound infection, including but not limited to, an antibiotic, anti-microbial agent, and an anti-bacterial agent. The collagen biofabric comprised in the invention has clinical and therapeutic utility in the treatment of wound infections from any microorganism known in the art, *e.g*., microorganisms that infect wounds originating from within the human body, which is a known reservoir for pathogenic organisms, or from environmental origin. A non-limiting example of the microorganisms, the growth of which in wounds may be reduced or prevented by the methods and compositions of the invention are *S. aureus, St. epidermis,* beta haemolytic *Streptococci, E. coli*, *Klebsiella* and *Pseudomonas* species, and among the anaerobic bacteria, the *Clostridium welchii* or *tartium,* which are the cause of gas gangrene, mainly in deep traumatic wounds.

As disclosed herein, the collagen biofabric comprised in the invention is used for wound treatment, including but not limited to epidermal wounds, skin wounds, chronic wounds, acute wounds, external wounds, internal wounds (e.g., the collagen biofabric may be wrapped around an anastosmosis site during surgery to prevent leakage of blood from suture lines, and to prevent the body from forming adhesions to the suture material), congenital wounds (e.g., dystrophic epidermolysis bullosa). In particular, the collagen biofabric has enhanced utility in the treatment of pressure ulcers (e.g., decubitus ulcers). Pressure ulcers occur frequently with patients subject to prolonged bedrest, e.g., quadriplegics and paraplegics who suffer skin loss due to the effects of localized pressure. The resulting pressure sores exhibit dermal erosion and loss of the epidermis and skin appendages.

The collagen biofabric comprised in the invention may also be used in the treatment of burns, including but not limited to first-degree burns, second-degree bums (partial thickness bums), third degree bums (full thickness bums), infection of burn wounds, infection of excised and unexcised burn wounds, infection of grafted wound, infection of donor site, loss of epithelium from a previously grafted or healed burn wound or skin graft donor site, and burn wound impetigo.

### 5.4.1.3 TISSUE ENGINEERING

Disclosed herein is the use of the collagen biofabric comprised in the invention as a vehicle for the transportation of cultured skin cells, *e.g.,* as a support for epithelial growth and cases differentiation. In certain cases, to populate the biofabric with cells for forming tissue and/or organoids (*i*.*e*., resembling in superficial appearance or in structure any of the organs or glands of the body), the biofabric can be treated with cellular adhesion factors to enhance attachment of cells to the biofabric during the process of repopulating the biofabric cases with such new cells. In certain cases, the extent of attachment of cells is increased by treating the amniotic membrane with serum, e.g., human or fetal bovine serum). In other embodiments, the extent of attachment of cells is increased by treating the amniotic membrane with fibronectin.

The collagen biofabric comprised in the invention may be used in guided tissue regeneration techniques *e*.*g*., to regenerate or replace diseased or damaged tissue. Disclosed herein is the use of the biofabric of the invention by directly implanting the biofabric at the site of treatment or by the formation of a prosthetic device. The collagen biofabric of the invention is particularly useful in any situation, such as following surgery especially oral or dental surgery (as described in more detail in Section 5.4.2.2), where enhanced wound healing and/or replacement of dermis is desirable. The utility of the collagen biofabric comprised in the invention in guided tissue regeneration is due in part to its ability to provide conditions which prevent ingrowth of other tissues into the area where regeneration is required.

For example, where a substantial portion of a tooth root is removed due to decay or disease, it is desirable that healthy bone regeneration occurs to replace the bone tissue removed. However, it has been found that the cavity left by removal of the bone is quickly filled by connective tissue and that this ingrowth of connective tissue effectively prevents bone regeneration. To prevent such ingrowth, the collagen biofabric comprised in the invention can be surgically inserted around the periphery of the wound cavity. The biofabric prevents or hinders the invasion of the wound cavity by unwanted cell types and thus allows the preferred cells to grow into the cavity, thereby healing the wound.

As disclosed herein, the collagen biofabric has utility as a transplant for ocular surface reconstruction for example in a subject with Stevens Johnson syndrome, limbal stem cell deficiency secondary to a chemical burn, or with chemical and/or thermal burns. The biofabric comprised in the invention is expected to have enhanced clinical utility for example by promoting epithelialization. The biofabric of the invention is expected to have an enhanced clinical utility relative to the amniotic membranes used in the art for tissue engineering purposes, *see, e.g*., Gomes et al., 2003, Opthalmology, 119: 166-73; Ti et al., 2001, Opthalmology, 108: 1209-1217; Meller et al., 2000, Ophthalmology, 107: 980-9; Gris et al., 2002 Opthalmology, 109: 508-12; Koizumi et al., 2000, Invest. Opthal. and Visual Science, 41: 2506-13; Pires et al., 1999, Arch. Opthalmol. 117: 1291-7; Tseng et al., 1998, Arch. Opthalmol. 116:431:441; Heiligenhaus et al., 2001 Invest. Opthal. and Visual Science 42: 1969-74; Anderson et al. 2001, Br. J. Opthalmol. 85: 567-75.

Disclosed herein is populating the collagen biofabric with living cells, including but not limited to adult tissue cells, autologous cells, and stem cells. The stem cells for use in the methods of the invention can be totipotent, pluripotent, or differentiated tissue specific cells. Stem cells for use in the methods of the invention can be obtained by standard methods known to one skilled in the art. Preferably, stem cells are collected according to the method disclosed in U.S. Application No. 10/74,976, filed February 13, 2002. The cells are not of human embryonic origin.

Disclosed herein is the use of the collagen biofabric comprised in the invention (e.g., as three dimensional scaffolds) for the development of bioengineered tissue and organoids, including but not limited, to blood vessels, heart valves, liver, pancreas, and ligaments. Although not intending to be bound by any mechanism of action, the utility of the biofabric comprised in the invention as a bioengineered tissue is due in part, to its hemostatic property in promoting blood coagulation. The biofabric comprised in the invention is particularly useful for comprised in vascular prostheses and as a transplant in vessel surgery. The biofabric comprised in the invention can be used, e.g., as a circumferential covering over the anastomotic sites of blood vessels (or vessels to grafts) during vascular surgery procedures to prevent leakage of blood from the suture lines and prevent the body from forming adhesions to the suture material.

### 5.4.2 METHODS OF USE OF THE COLLAGEN BIOFABRICS IN SURGICAL PROCEDURES

Disclosed herein is the use of the collagen biofabric comprised in the invention as a surgical graft. Disclosed herein is a surgical graft comprising a collagen biofabric comprised in the invention or a laminate thereof. Disclosed herein are also methods of preparing and using the surgical graft.

Disclosed herein is a method of using the surgical graft in a surgical procedure, so that the surgical graft is applied directly to the surgical site of the subject, e.g., an internal site, or an external site.

The collagen biofabric is used as a surgical graft during a surgical procedure as disclosed in more detail below, for example, to prevent leakage of blood from suture lines and to prevent the body from forming adhesions to the suture materials. The surgical graft is used as a covering over the anastosomotic sites, e.g., of the GI tract during GI surgery to prevent leakage of intestinal fluid and bile from the suture lines and to prevent the body from forming adhesions to the suture materials.

Disclosure herein is using the biofabric as a graft or dressing to cover burned or surgical skin wounds; to prevent adhesion in all intra peritoneal surgeries or other reconstruction on the serosal surfaces covering the abdomen, chest cavity and pericardium; to reconstruct all mocosal surfaces lining the oral and nasal cavities, respiratory tracts, gastrointestinal tracts, and urogenital tracts; as a substrate to support dural repair in brain surgeries; as a substrate to promote nerve regeneration in the central and peripheral nervous systems; and to reconstruct soft tissues to prevent adhesion in joint or tendon repairs.

Disclosed herein is impregnating the surgical graft with one or more biomolecule, preferably a therapeutic agent, depending on the particular intended surgical use. Such biomolecules include but are not limited to, antibiotics (such as Clindamycin, Minocycline, Doxycycline, Gentamycin), hormones, growth factors, anti-tumor agents, anti-fungal agents, anti-viral agents, pain medications, anti-histamines, anti-inflammatory agents, anti-infectives including but not limited to silver (such as silver salts, including but not limited to silver nitrate and silver sulfadiazine), elemental silver, antibiotics, bactericidal enzymes (such as lysozome), wound healing agents (such as cytokines including but not limited to PDGF, TGF; thymosin), Hyaluronic acid as a wound healing agent, wound sealants (such as fibrin with or without thrombin), cellular attractant and scaffolding reagents (such as fibronectin) and the like. In a specific example, the collagen biofabric may be impregnated with at least one growth factor, for example, fibroblast growth factor, epithelial growth factor, *etc.* The biofabric may also be impregnated with small organic molecules such as specific inhibitors of particular biochemical processes e.g., membrane receptor inhibitors, kinase inhibitors, growth inhibitors, anticancer drugs, antibiotics, *etc.*

Disclosed herein is populating the surgical graft with living cells, including but not limited to, stem cells, totipotent stem cells, pluripotent stem cells, multipotent stem cells, tissue specifc stem cells, embryonic like stem cells, committed progenitor cells, fibroblastoid cells. In other embodiments, the invention encompasses populating the surgical graft of the invention with specific classes of progenitor cells including but not limited to chondrocytes, hepatocytes, hematopoietic cells, pancreatic parenchymal cells, neuroblasts, and muscle progenitor cells. The cells are not of human embryonic origin.

### 5.4.2.1 OPTHALMOLOGY

The collagen biofabric comprised in the invention has clinical and therapeutic utility in the treatment of an eye related disease or disorder. The collagen biofabric comprised in the invention is particularly useful for the treatment and/or prevention of ocular surface diseases including but not limited to, corneal ulcerations/perforations, bullous keratopathy, ocular dermoids/tumors, primary pterygium, persistent corneal epithelial defect, acute and chronic alkali bums, thermal bums, aniridia, atopic keratitis, idiopathic limbal stem cell deficiency, corneal pannus, neovascularization, rheumatoid corneal melt, ocular cicatricial pemphigoid, leaking filtering bleb, exposed Ahmed valve tube, Serratia cellulitis with subsequent symblepharon, acute and chronic Stevenson Johnson syndrome. The collagen biofabric comprised in the invention is particularly effective in promoting healing of persistent corneal epithelial defects with ulceration; promoting epithelialization; facilitation of growth of epithelial and stem cells; which are not of human embryonic origin. reduction of inflamation and pain, inhibition of angiogenesis and scarring; restoration of the epithelial phenotype; and as a substrate alternative to conjunctival autograft during the "bare sclera" removal of pterygia.

Disclosed herein is transplantation using the collagen biofabric comprised in the invention for the treatment of symptomatic bullous keratopathy, preferably in humans, most preferably in humans with poor visual potential. The biofabric comprised in the invention is expected ot have an enhanced therapeutic and clinical utility relative to other standard procedures used in the art for the treatment of symptomatic bullous keratopathy, specifically, conjunctival flap construction. The advantage of transplantation using the biofabric comprised in the invention over standard procedures used in the art for the treatment of symptomatic bullous keratopathy, specifically, in conjunctival flap construction, include for example, reduction of pain, ease of performance, a more cosmetically acceptable appearance, and a reduction in complications such as ptosis and limbal stem cell deficiency. The collagen biofabric comprised in the invention provides an improved alternative to conjunctival flaps for promoting healing of corneal epithelial defects with ulceration; an improved method for conjunctival surface reconstruction for symbelpharon lysis; an improved method for surgical removal of tumors, lesions, or scar tissue from the conjunctival or corneal surface; an improved method for glaucoma surgeries by correcting bleb leakage; an improved substrate alternative to conjunctival autograft during the "bare sclera" removal of pterygia; and an improved method for preventing recurrence of band keratopathy

Disclosed herein is the use of the biofabric as an ophthalmological surgical graft. Disclosure herein is the preparation of grafts comprising the biofabric comprised in the invention further comprising one or more therapeutic agents that can be delivered to the recipient when attached to the recipient. Examples of therapeutic agents that can be delivered using the biofabric comprised in the invention include but are not limited to pilocarpine, eryhtromycin, gentamicin, vancomycin, tobramycin, netilmycin, polymyxin B sulfate, trimethoprim, amphotericin B, anti-cancer agents, antibiotics, cyclosporin, *etc.*

The collagen biofabric comprised in the invention also has utility in reducing the corneal haze induced by excimer laser photerefractive/therapeutic keratectomy.

The collagen biofabric comprised in the invention for use in ophthalmic procedures may be provided in various configurations including but not limited to inserts, shields, particles, gels, aqueous injections, sponges, films.

Conventional surgical techniques for ophtalmic grafts, known to one skilled in the art are disclosed herein.

An exemplary protocol for use of a collagen biofabric comprised in the invention as an ophthalmic surgical graft may comprise the following steps: the diseased corneal and/or conjunctival tissues are removed; the surgical graft, prepared in accordance with the methods disclosed herein is provided under sterile conditions, and trimmed by a free hand technique to cover the corneal epithelium or bare sclera. Using a suture, the graft is anchored to the junction of the bare sclera and conjunctiva; cardinal sutures are placed; and then interrupted sutures are used to create an even distribution of the tension over the graft's surface; so that any space is avoided between the graft and the recipient sclera or cornea. At the junction of the graft and the host, the free edge of the graft must remain under the recipient conjunctival membrane to allow sliding of the host epithelium over the basal lamina at the point of junction with the donor tissue, otherwise the graft is extruded by the recipient.

### 5.4.2.2 DENTAL

The collagen biofabric comprised in the invention has particular utility in dentistry, *e.g.,* periodontal surgery, guided tissue regeneration for regeneration of periodontal tissue, guided bone regeneration, and root coverage. Disclose herein is the use of the collagen biofabric comprised in the invention to promote regeneration of periodontal intrabony defects, including but not limited to matched bilateral periodontol defects, interdental intrabony defects, deep 3-wall intrabony defects, 2-wall intrabony defects, and intrabony defects 2 and 3. The collagen biofabric comprised in the invention is expected to have an enhanced therapeutic utility and enhanced clinical parameters for the treatment of periodontal intrabony defects relative to other techniques known in the art, *e.g.,* use of cross-linked collagen membranes such as those disclosed in Quteish et al., 1992, J. Clin. Periodontol. 19(7): 476-84; Chung et al., 1990, J. Periodontol. 61(12): 732-6; Mattson et al., 1995, J. Periodontol. 66(7): 635-45; Benque et al., 1997, J. Clin. Periodontol. 24(8): 544-9; Mattson et al., 1999, J. Periodontol. 70(5): 510-7) Examples of clinical parameters that are improved using the collagen biofabric comprised in the invention include but are not limited to plaque and gingival index scorings, probing pocket depth, probing attachment depth, and classification of furcation involvement and bony defect, which are known to one skilled in the art.

Disclosed herein is the use of the biofabric comprised in the invention in treating class II furcation defects including but not limited to bilateral defects, paired buccal Class II mandibular molar furcation defects, and bilateral mandibular furcation defect. The utility of the collagen biofabric comprised in the invention in treating class II furcation defects can be explained in part by its ability to regenerate lost periodontium in furcation defects. The biofabric comprised in the invention is expected to have an enhanced therapeutic and clinical utility relative to the collagen membranes used in the art for the treatment of class II furcation defects, such as those disclosed in Paul et al., 1992, Int. J. Periodontics Restorative Dent. 12: 123-31; Wang et al., 1994, J. Periodontol. 65: 1029-36; Blumenthal, 1993, J. Periodontol. 64: 925-33; Black et al., 1994, J. Periodontol. 54: 598-604; Yukna et al., 1995, J. Periodontol. 67: 650-7).

Disclosed herein is also the use of the biofabric comprised in the invention in root coverage procedures. The utility of the biofabric comprised in the invention in root coverage can be explained in part due to its ability to replace lost, damaged or disease gingival tissue based on the principles of guided tissue regeneration. The biofabric comprised in the invention is expected to have an enhanced clinical utility in root coverage as compared to collagen membranes in the art traditionally used for root coverage such as those disclosed in Shieh et al., 1997 J. Periodontol., 68: 770-8; Zahedi et al., 1998 J. Periodontol. 69: 975-81; Ozcan et al., 1997 J. Marmara Univ. Dent. Fa. 2: 588-98; Wang et al., 1997 J. Dent. Res. 78(Spec Issue): 119(Abstr. 106), for reasons cited *supra.*

Disclosed herein is also the use of the collagen biofabric in a subject with a periodontal disease including but not limited to, periodontitis and gingivitis. The biofabric comprised in the invention also has clinical utility as an adjunct to scaling and root planning procedures. Disclosed herein is treating a subject with a periodontal disease using a collagen biofabric comprised in the invention. An exemplary method for treating a periodontal disease in a subject with using a collagen biofabric comprised in the invention comprises inserting a collagen biofabric, which is preferably impregnated with an antibiotic such as chlorhexidine gluconate, into one or more periodontal pockets in the subject, e.g., greater than or equal to 5mm. Preferably the collagen biofabric is biodegradable.

The collagen biofabric comprised in the invention for use in dentistry may be impregnated with one or more biomolecules depending on the type of dental disorder being treated. Any biomolecule known in the art for the treatment of dental disorders is encompassed in the methods and compositions of the invention. As disclosed herein, the collagen biofabric used in the treatment of a dental disorder associated with an infection may be impreganted with one or more antibiotics, including but not limited to doxocyclin, tetracyclin, chlorhexidine gluconate, and minocycline.

### 5.4.2.3 NEUROLOGY

The collagen biofabric comprised in the invention is also useful in repairing injured nerves, particularly in repairing severed peripheral nerves, and neurosurgical procedures. Disclosed herein is the use of the collagen biofabric, for example, in dural replacements and in peripheral nerve repair. The collagen biofabric comprised in the invention has enhanced clinical utility as a dural substitute or in nerve repair in contrast to other methods used in the art, *e.g*., Berger et al., 1970, Acta. Neurochir. 23: 141; Ducker et al., 1968, Mil. Med. 133: 298; U.S. Patent Nos. 4,778,467; 4,883,618; 3,961,805; 5,354,305, for reasons set forth above.

The collagen biofabric comprised in the invention may be used as a prostheses around nerve anastosmosis, for example it can be used to wrap peripheral nerve anastosmosis. The collagen biofabric comprised in the invention has particular utility in nerve repair, for example, by promoting the formation of a longitudinal connective tissue framework across the area of repair and thus giving rise to a longitudinally pattern of sheath cell and axonal regeneration.

Repair of peripheral nerves is commonly done using sutures in a procedure known as nerorraphy *(*Jennings et al., 1955, Surgery: 206). However, this approach has had limited success since the methods of suturing severed nerves is difficult. The collagen biofabric comprised in the invention may thus provide an alternative to a sutureless method of nerve repair, whereby, for example, the nerve ends are enclosed in a tubular prosthetic device comprising of the biofabric of the invention., and thus bringing the severed ends in close proximity for regeneration.

### 5.4.2.4 UROLOGY

The collagen biofabric comprised in the invention has particular utility in the correction of urinary incontinence. Urinary incontinence results from failure of the urethra to remain closed during storage. The cause may be extrinsic, *e.g*., poor anatomic support of the urethra and bladder neck results in incontinence and responds to pelvic floor resuspension. Urethral failure, alternatively may be intrinsic, *i.e.,* poor urethral function. Traditionally, urinary incontinence is corrected using bulking agents such as collagen, fat, silicone *(See,* review Lightner, 2002, Current Opinion in Urology, 12(4): 333-8). The collagen biofabric comprised in the invention has enhanced utility, i.e., improves continence, relative to the bulking agents described in the art. Further the collagen biofabric comprised in the invention has enhanced therapeutic utility, e.g., reduced local complications, reduced urinary tract infection, no host reaction, reliable durability, and greater safety.

The utility of the collagen biofabric comprised in the invention in correcting urinary incontinence is due in part to the physical features unique to the collagen biofabric as described herein, particularly, its non-immunogenicity. As disclosed herein, the collagen biofabric is used as an implant, *e.g.,* as a urethral implant. Although not intending to be bound by any particular theory, the collagen biofabric comprised in the invention may have enhances therapeutic utility by increasing, for example, urethral closure pressure and resistance to passive outflow of urine.

### 5.4.2.5 ORTHOPEDICS

The collagen biofabric comprised in the invention may be used in orthopedic surgical procedures. As disclosed herein the collagen biofabric may be used for orthopedic defects, e.g., acquired or congenital defects. Reconstruction of local defects resulting from trauma or surgical resurrection of a bone tumor is a major problem in orthopedic or maxillofacial surgery. Typically, synthetic bone substitutes or collagenous membranes have been used due in part to their osteoinductive activities *(See, e.g.,* Rao et al., 1995, J. Biomater. Sci. Polymer Edn. 7(7): 623-45). However, a common problem has been a systemic infection as a result of the implanted material. The collagen biofabric comprised in the invention, however, is advantageous over the material used in the art, particularly due to its low immunogenicity as a bone substitute for use in orthopedic defects. As disclosed herein , the collagen biofabric may be used as a prosthesis for reconstructing tendons, ligaments and cartilage. As disclosed herein, the collagen biofabric comprised in the invention may be used as a prosthesis as a bone replacement.

### 5.4.2.6 CARDIOVASCULAR SURGERY

The collagen biofabric comprised in the invention may be used in cardiovascular surgical procedures, for example, as a prostheses for constructing large and small vessels; for repairing congenital malformations of vessels and diseased valves.

The biofabric comprised in the invention has particular utility as a transplant in vessel surgery, e.g., venous or arterial transplant. The utility of the biofabric in vessel surgery is due, in part, to its non-toxicity, non-immunogenicity; stability; ease of handling; anti-thrombogenic characteristic; minimal implantation porosity of the vessel wall; availability in various sizes; reproducibility of the material. As disclosed herein, the biofabric comprised in the invention may be used as a valve replacement.

### 5.4.2.7 DRUG DELIVERY

The collagen biofabric comprised in the invention can be used as a drug delivery vehicle for controlled delivery of a drug, *e.g.,* a therapeutic agent. In some embodiments the collagen biofabric delivers the one or more therapeutic agents to a subject, preferably a human. The therapeutic agents encompassed within the scope of the invention are proteins, peptides, polysaccharides, polysaccharide conjugates, genetic based vaccines, live attenuated vaccines, whole cells. A non-limiting example of drugs for use in the methods of the invention is antibiotics, anti-cancer agents, anti-bacterial agents, anti-viral agents; vaccines; anesthetics; analgesics; anti-asthmatic agents; anti-inflammatory agents; anti-depressants; anti-arthritic agents; anti-diabetic agents; anti-psychotics; central nervous system stimulants; hormones; immuno-suppressants; muscle relaxants; prostaglandins.

The collagen biofabric may be used as a delivery vehicle for controlled delivery of one or more small molecules to a subject, preferably a human. As disclosed herein the collagen biofabric delivers the one or more small molecules to a subject, preferably a human. As used herein, the term "small molecule," and analogous terms, include, but are not limited to, peptides, peptidomimetics, amino acids, amino acid analogs, polynucleotides, polynucleotide analogs, nucleotides, nucleotide analogs, organic or inorganic compounds (*i.e*,. including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, organic or inorganic compounds having a molecular weight less than about 100 grams per mole, and salts, esters, and other pharmaceutically acceptable forms of such compounds. Salts, esters, and other pharmaceutically acceptable forms of such compounds are also encompassed.

As disclosed herein , the collagen biofabric comprised in the invention as a vehicle for drug delivery results in enhanced absorption of the drug; improved pharmacokinetic profile, and systemic distribution of the drug relative to the other drug delivery systems known in the art. By improved pharmacokinetics it is meant that an enhancement of pharmacokinetic profile is achieved as measured, for example, by standard pharmacokinetic parameters such as time to achieve maximal plasma concentration (Tₘₐₓ); magnitude of maximal plasma concentration (Cₘₐₓ); time to elicit a detectable blood or plasma concentration (T_{lag}). By enhanced absorption it is meant that absorption of the drug is improved as measured by such parameters. The measurement of pharmacokinetic parameters are routinely performed in the art.

### 6. EXAMPLES

### 6.1 METHOD OF PRODUCING THE COLLAGEN BIOFABRIC

### MATERIALS

The following materials were used in preparation of the collagen biofabric.

### Materials/Equipment

- Copy of Delivery Record
- Copy of Material/Family Health History/Informed Consent
- Source Bar Code Label (Donor ID number)
- Collection # (A sequential number is assigned to incoming material)
- Tissue Processing Record (Document ID #ANT-19F); a detailed record of processing of each lot number is maintained
- Human Placenta (less than 48 hours old at the start of processing)
- Sterile Surgical Clamps/Hemostats
- Sterile Scissors
- Sterile Scalpels
- Sterile Cell Scraper (Nalgene NUNC Int. R0896)
- Sterile Gauze (non-sterile PSS 4416, sterilized)
- Sterile Rinsing Stainless Steel Trays
- Disinfected Processing Stainless Steel Trays
- Disinfected Plastic Bin
- Sterile 0.9% NaCl Solution (Baxter 2F7124)
- Sterile Water (Milli Q plus 09195 or Baxter 2F7113)
- Sterile Specimen Containers (VWR 15704-014)
- Personal Protective Equipment (including sterile and non-sterile gloves)
- Certified Clean Room
- Previously Prepared Decellularizing Solution (D-cell); 0.01-1% deoxycholic acid sodium monohydrate
- Disinfected Bin
- Rocking Platform (VWR Model 100)
- Timer (VWR 21376890)
- Disinfected Plastic Frame Mesh
- PVC Wrap Film
- Vacuum Pump (Schuco-Vac 5711-130)
- Gel Dryer (*i.e*., heat dryer; BioRad Model 583)
- Disinfected Stainless Steel Cutting Board
- Pouches for Packaging
- Sterile Stainless Steel Ruler (General Tools MFG. Co 1201)
- Traceable Digital Thermometer (Model 61161-364, Control Company)
- Accu-Seal Automatic Sealer (Accu-Seal, Model 630-1B6)

The expectant mother was screened at the time of birth for communicable diseases such as HIV, HBV, HCV, HTLV, Syphilis, CMV and other viral, and other pathogens that could contaminate the placental tissues being collected. Only tissues collected from donors whose mothers tested negative or non-reactive to the above-mentioned pathogens were used to produce the collagen biofabric.

Following normal birth, the placenta, umbilical cord and umbilical cord blood were spontaneously expelled from the contracting uterus. The placenta, umbilical cord, and umbilical cord blood were collected following birth. The materials were transported to the laboratory where they were processed under aseptic conditions in a Clean room having a HEPA filtration system, which was turned on at least one hour prior to processing. Gloves (sterile or non-sterile, as appropriate) were worn at all times while handling the product. All unused (waste) segments of the amnion/chorion and contaminated liquids, generated during tissue processing were disposed of as soon as feasible.

### STEP I.

A *sterile field* was set up with sterile Steri-Wrap sheets and the following instruments and accessories for processing were placed on it.
- sterile tray pack
- sterile Cell Scraper
- sterile scalpel
- disinfected processing tray

Sterile pack ID # was recorded in the Processing Record.

The placenta was removed from the transport container and placed onto the disinfected stainless steel tray. Using surgical clamps and scissors, the umbilical cord was cut off approximately 2 inches from the placental disc. The umbilical cord was placed into a separate sterile container for further processing. The container was labeled with Tissue ID Bar Code; and the material and storage solution(s) present (e.g., type of media) were identified. In some cases, the umbilical cord was discarded if not requested for other projects.

Starting from the edge of the placental membrane, the amnion was separated from the chorion using blunt dissection with fingers. This was done prior to cutting the membrane.

After the amnion was separated from the entire surface of the chorion and placental disc, the amniotic membrane was cut around the umbilical cord stump with scissors and detached from the placental disc. In some instances, if the separation of the amnion and chorion was not possible without tearing the tissue, the amnion and chorion were cut from the placental disc as one piece and then peeled apart.

The chorion was placed into a separate specimen container to be utilized for other projects. The container was labeled with the Tissue ID Bar Code, the material and storage solution(s) present (*e*.*g*., type of media) were identified, initialed and dated.

If any piece of amnion was still attached to the placental disc it was peeled from the disc and cutting off around the umbilical cord with scissors. The placenta was placed back into the transport container to be utilized for other projects.

The appropriate data was recorded in the Tissue Processing Record.

The amniotic membrane was kept in the tray with sterile 0.9% NaCl solution. Preferably, the amniotic membrane is stored by refrigeration for a maximum of 72 hours from the time of delivery prior to the next step in the process.

### STEP II.

The amniotic membrane was removed from the specimen container one piece at a time and placed onto the disinfected stainless steel tray. Other pieces were placed into a separate sterile stainless steel tray filled with sterile water until they were ready to be cleaned. Extra pieces of amnion from the processing tray were removed and placed in a separate rinsing stainless steel tray filled with sterile water.

The amniotic membrane was rinsed with sterile water if grossly contaminated with blood maternal or fetal fluids/materials changing sterile water as needed.

The amniotic membrane was placed on the processing tray with the maternal side facing upward. Using a sterile Cell Scraper, as much as possible of visible contamination and cellular material from the maternal side of the amnion was carefully removed. (Note: minimal pressure should be applied for this step to prevent tearing the membrane). Sterile water was used to aid in the removal of cells and cellular debris. The amniotic membrane was further rinsed with sterile water in the separate sterile stainless steel rinsing tray.

The amniotic membrane was turned over so that the fetal side was facing upward and placed back on the processing tray and rinsed with sterile water. Visible cellular material and debris using the Cell Scraper was gently removed (Note: minimal pressure should be applied for this step to prevent tearing the membrane). Sterile water was used to aid in the removal of cells and cellular debris.

The amniotic membrane was rinsed with sterile water in between cleaning rounds in separate sterile rinsing trays. The tissue was cleaned as many times (cleaning rounds) as necessary to remove most if not all of visible cellular material and debris from both sides of the membrane. The sterile water was changed in the rinsing trays in between rinses.

The processing tray was rinsed with sterile water after each cleaning round.

All other pieces of amnion were processed in the same manner and placed into the same container. Tissue Id Bar Code was affixed, the material and storage solution(s) present (e.g., type of media) were identified, initials date were added.

The appropriate information and the date were recorded in the Tissue Processing Record.

### STEP III.

The amniotic membrane was removed from the rinsing tray, (or from storage container) excess fluid was gently squeezed out with fingers and the membrane was placed into the sterile specimen container. The container was filled up to the 150 ml mark with D-cell solution ensuring that all of the amniotic membrane was covered and the container was closed.

The container was placed in the bin on the rocking platform. The rocking platform was turned on and the membrane was agitated in D-cell solution for a minimum of 15 minutes and a maximum of 120 minutes at Setting #6.

A new sterile field was set up with new sterile instruments and disinfected tray in a same manner as in the Step I. Sterile pack ID # was recorded in the Processing Record.

After agitation was completed, the rocking platform was turned off and the membrane was removed from the container. The membrane was placed into a new sterile stainless steel processing tray. Sterile 0.9% NaCl solution was added to cover the bottom of the tray.

Using a new sterile Cell Scraper, residual D-cell and cellular material (if any) was removed from both sides of the tissue. This step was repeated as many times as needed to remove as much as possible of visible residual cellular material from the entire surface on both sides. The membrane was rinsed with sterile 0.9% NaCl solution in a separate rinsing tray in between cleaning rounds. The sterile 0.9% NaCl solution was changed in the rinsing trays in between rinses.

After the last cleaning round was completed, the membrane was rinsed with sterile 0.9% NaCl solution and placed into the new sterile specimen container filled with sterile 0.9% NaCl solution.

All remaining pieces of amniotic membrane were processed in exactly the same manner.

When all amniotic membrane pieces were processed and in the container with the sterile 0.9% NaCl solution, the container was placed in the bin on the rocking platform to agitate for a minimum of 5 minutes at setting #6. After agitation was completed, the membrane was removed from the specimen container, the sterile 0.9% NaCl solution was changed in the container and the membrane was placed back into the specimen container.

The specimen container was labeled with Tissue ID Bar Code and Quarantine label. The material and storage solution(s) present (*e*.*g*., type of media) were identified, initialed and dated. The specimen container was placed into a clean zip-lock bag and placed in the refrigerator (2 - 8°C).

All appropriate data was recorded in the Tissue Processing Record.

When serology results became available, the appropriate label (Serology Negative or For Research Use Only) was placed on the top of the Quarantine label and those containers were segregated from Quarantined ones.

### STEP IV.

Before proceeding with Step IV, the Tissue Status Review was checked to make sure all applicable test results were negative.

A sterile field was set up with sterile Steri-Wrap sheet and all sterile and disinfected instruments and accessories were set up in the same manner as in Steps II and III.

The membrane was removed from the refrigerator and placed into a new sterile stainless steel processing tray. Sterile 0.9% NaCl solution was added to cover the bottom of the tray.

All visible cellular material and debris (if any) was gently removed using a new sterile Cell Scraper (Note: minimal pressure should be applied for this step to prevent tearing the membrane). Sterile 0.9% NaCl solution was used to aid in removal of the cells and debris.

The membrane was rinsed in the separate sterile stainless steel rinsing tray filled with the sterile 0.9% NaCl Solution. 0.9% NaCl Solution was changed in between cleaning rounds. The membrane was placed into a new sterile specimen container, the container was filled with fresh sterile 0.9% NaCl solution and placed on the rocking platform for agitation for a minimum of 5 minutes at Setting #6.

The previous step was repeated 3 times and the sterile 0.9% NaCl solution was changed in between each agitation. Appropriate data was recorded in the Tissue Processing Record.

The membrane was removed from the specimen container one piece at a time, excess fluid was gently squeezed out with fingers and the membrane was placed onto a sterile processing tray. The membrane was gently stretched until flat; ensuring it was fetal side down.

The frame was prepared by cutting the disinfected plastic sheet with sterile scissors. The size of the frame should be approximately 0.5 cm smaller in each direction than the membrane segment. The frame was rinsed in the rinsing tray filled with sterile 0.9% NaCl solution.

The frame was placed on the slightly stretched membrane surface and pressed on it gently. It is imperative that the smooth side of the plastic frame faces the tissue.

Using a scalpel, the membrane was cut around the frame leaving approximately 0.5 cm extending beyond frame edges. The excess membrane was placed back into the specimen container

The membrane edges that are extended beyond the frame were wrapped over the edges of the frame using clamps or tweezers and put aside on the same tray.

The next piece of membrane was processed in the same manner. It is important the total area to be dried does not exceed 300 cm² per heat dryer. While 'framing out' the piece of membrane, the non-framed pieces should remain in the container in sterile 0.9% NaCl solution.

The drying temperatures of dryers were set and verified using a calibrated digital thermometer with extended probe. The drying temperature was set at 50°C. The data was recorded in the Tissue Processing Record.

The vacuum pump was turned on.

A sterile gauze was placed on the drying platform of the heat dryer, covering an area slightly larger than the area of the framed membrane. It is important to make sure that the total thickness of the gauze layer does not exceed thickness of one folded 4 x 4 gauze.

One sheet of plastic framing mesh was placed on top of the gauze. The plastic mesh edges should extend approximately 0.5 - 1.0 cm beyond gauze edges.

The framed membrane was gently lifted and placed on the heat dryer platform on top of the plastic mesh with the membrane side facing upward. This was repeated until the maximum amount of membrane (without exceeding 300 cm²) was on the heat dryer platform. (NOTE: fetal side of the amnion is facing up).

A piece of PVC wrap film was cut large enough to cover the entire drying platform of the heat dryer plus an extra foot.

With the vacuum pump running, the entire drying platform of the heat dryer was gently covered with the plastic film leaving ½ foot extending beyond drying platform edges on both sides. Care was taken that the film pull tightly against the membrane and frame sheet *(i.e.,* it is "sucked in" by the vacuum) and that there were no air leaks and no wrinkles over the tissue area). The lid was subsequently closed.

The vacuum pump was set to approximately -22 inches Hg of vacuum. The pump gage was recorded after 2-3 min of drying cycle. The membrane was heat vacuum dried for approximately 60 minutes. Approximately 15 - 30 minutes into the drying process, the sterile gauze layer was replaced in the heat dryer with a new one. The total thickness of the gauze layer must not exceed thickness of one folded 4 x 4 gauze.

After the change, care was taken so that the plastic film pulled tightly against the membrane and the frame sheet and there were no air leaks and no wrinkles over the membrane area.

The integrity of the vacuum seal was periodically checked by checking the pump pressure monometer. After completion of the drying process, the heat dryer was opened and the membrane was cooled down for approximately two minutes with the pump running.

A new sterile field was set up with sterile Steri-wrap and disinfected stainless steel cutting board underneath it. As this point sterile gloves were used. With the pump still running, the plastic film was gently removed from the membrane sheet starting at the corner and holding the membrane sheet down with a gloved hand. The frame was gently lifted with the membrane off the drying platform and placed on the sterile field on the top of the disinfected stainless steel cutting board with the membrane side facing upward. Using a scalpel, the membrane sheet was cut through making an incision along the edge 1 -2 mm away from the edge of the frame. The membrane was held in place with a gloved (sterile glove) hand. Gently the membrane sheet was lifted off of the frame by peeling it off slowly and then placed on the sterile field on the cutting board.

Using scalpel or sharp scissors, the membrane sheet was cut into segments of specified size. All pieces were cut and secured on the sterile field before packaging. A single piece of membrane was placed inside the inner peel-pouch package with one hand (sterile) while holding the pouch with another hand (non-sterile). Care was taken not to touch pouches with 'sterile' hand. After all pieces were inside the inner pouches they were sealed. A label was affixed with the appropriate information (*e*.*g*., Part #, Lot #, *etc.)* in the designated area on the outside of the pouch. All pieces of membrane were processed in the same manner. The labeled and sealed peel-pouch packages were placed in the waterproof zip-lock bag for storage until they were ready to be shipped to the sterilization facility or distributor. All appropriate data were recorded on the Tissue Processing Record.

### 6.2 EVALUATION OF THE COLLAGEN BIOFABRIC FOR HYDRATION/SUTURABILITY

In order to generate qualitative and quantitative feedback regarding the surgical handling, hydration periods and suturability of the biofabrics comprised in the present invention, amniotic membrane samples prepared according to the methods of the present invention are provided to four experienced, well respected ocular surface surgeons for evaluation. The samples are evaluated by the surgeons by performing tissue grafts on pig eye specimens to determine surgical handling properties and suturability of the biofabrics comprised in the invention.

The following methodology may be used by each surgeon:
(1) The biofabric is cut dried to fit a single quadrant of the pig's eye;
(2) The cut biofabric is placed on the surface of the pig's eye;
(3) The biofabric is hydrated with sterile saline solution and the graft is allowed to activate, *i*.*e*., re-hydrate, on the pig's eye for hydration periods of 2, 5, 10, and 20 minutes;
(4) The biofabric is sutured to the epithelium of the pig's eye with several 9-0 vicryl suture bites; and
(5) The surgeons make qualitative notes regarding tissue quality, consistency, and suturability of the hydrated amniotic membrane

## Claims

1. A method of preparing an amniotic membrane laminate from a placenta having an amniotic membrane and a chorionic membrane comprising:
(a) separating the amniotic membrane from the chorionic membrane;
(b) decellularizing the amniotic membrane; and
(c) layering at least two of the decellularized amniotic membranes in contact with each other so that an amniotic membrane laminate is formed,
wherein the decellularization of the amniotic membrane does not constitute freezing at any point in preparation of the amniotic membrane or contacting the membrane with a protease.

2. The method of claim 1, further comprising washing the decellularized amniotic membrane at least once after step (b) and before step (c).

3. The method of claim 1, further comprising drying the decellularized amniotic membrane laminate.

4. The method of claim 1, further comprising assembling at least two of said amniotic membrane laminates into a complex three dimensional scaffold.

5. An amniotic membrane laminate comprising at least two layers of a dehydrated, decellularized and substratefree amniotic membrane, wherein said amniotic membrane has a native tertiary and quaternary structure.

6. The amniotic membrane laminate of claim 5, wherein the amniotic membrane comprises collagen, elastin, and fibronectin.

7. The amniotic membrane laminate of claim 5, further comprising one or more hydrogel compositions.

8. The amniotic membrane laminate of claim 7, wherein the hydrogel composition comprises a polymer selected from the group consisting of polyvinyl alcohol, polyethylene glycol, hyaluronic acid, dextran, and derivatives or analogs thereof.

9. The amniotic membrane laminate of any of claims 5-8, wherein the laminate is shaped into a sheet, tube or microsphere.

## Patentansprüche

1. Verfahren zur Herstellung eines amniotischen Membranlaminats aus einer Plazenta mit einer amniotischen Membran und einer chorionischen Membran umfassend:
(a) Trennen der amniotischen Membran von der chorionischen Membran;
(b) Dezellularisieren der amniotischen Membran; und
(c) Legen von mindestens zwei der dezellularisierten amniotischen Membrane in Kontakt zueinander, so dass ein amniotisches Membranlaminat gebildet wird,
wobei die Dezellularisierung der amniotischen Membran kein Einfrieren zu irgendeinem Zeitpunkt bei der Präparation der amniotischen Membran oder In-Kontakt-bringen der Membran mit einer Protease einsetzt.

2. Verfahren nach Anspruch 1, dass weiter das Waschen der dezellularisierten amniotischen Membran mindestens einmal nach Schritt (b) und vor Schritt (c) umfasst.

3. Verfahren nach Anspruch 1, dass weiter das Trocknen des dezellularisierten amniotischen Membranlaminats umfasst.

4. Verfahren nach Anspruch 1, dass weiter das Zusammenfügen von mindestens zwei der amniotischen Membranlaminate in ein komplexes dreidimensionales Gerüst umfasst.

5. Amniotisches Membranlaminat umfassend mindestens zwei Schichten einer dehydrierten, dezellularisierten und Substrat-freien amniotischen Membran, wobei die amniotische Membran eine natürliche tertiäre und quartäre Struktur hat.

6. Amniotisches Membranlaminat nach Anspruch 5, wobei die amniotische Membran Collagen, Elastin und Fibronectin umfasst.

7. Amniotisches Membranlaminat nach Anspruch 5, das weiter eine oder mehrere Hydrogelzusammensetzungen umfasst.

8. Amniotisches Membranlaminat nach Anspruch 7, wobei die Hydrogelzusammensetzung ein Polymer umfasst, dass aus der Gruppe ausgewählt wird bestehend aus Polyvinylalkohol, Polyethylenglykol, Hyaluronsäure, Dextran und Derivative oder Analoga davon.

9. Amniotisches Membranlaminat nach einem der Ansprüche 5-8, wobei das Laminat in die Form eines Bogens, einer Röhre oder einer Mikrosphäre gebracht ist.

## Revendications

1. Procédé de préparation d'un stratifié de membrane amniotique à partir d'un placenta ayant une membrane amniotique et une membrane chorionique consistant à :
(a) séparer la membrane amniotique de la membrane chorionique ;
(b) décellulariser la membrane amniotique : et
(c) disposer en couches au moins deux des membranes amniotiques décellularisées en contact l'une avec l'autre de telle manière qu'un stratifié de membrane amniotique est formé,
dans lequel la décellularisation de la membrane amniotique ne constitue pas de congélation en un point quelconque de la préparation de la membrane amniotique ou de mise en contact de la membrane avec une protéase.

2. Procédé de la revendication 1, consistant en outre à laver la membrane amniotique décellularisée au moins une fois après l'étape (b) et avant l'étape (c).

3. Procédé de la revendication 1, consistant en outre à sécher le stratifié de membrane amniotique décellularisée.

4. Procédé de la revendication 1, consistant en outre à assembler au moins deux desdits stratifiés de membrane amniotique dans un échafaudage complexe tridimensionnel.

5. Stratifié de membrane amniotique comprenant au moins deux couches d'une membrane amniotique déshydratée, décellularisée et sans substrat, dans lequel ladite membrane amniotique a une structure native tertiaire et quaternaire.

6. Stratifié de membrane amniotique de la revendication 5, dans lequel la membrane amniotique comprend du collagène, de l'élastine et de la fibronectine.

7. Stratifié de membrane amniotique de la revendication 5, comprenant en outre une ou plusieurs compositions d'hydrogel.

8. Stratifié de membrane amniotique de la revendication 7, dans lequel la composition d'hydrogel comprend un polymère choisi dans le groupe constitué de l'alcool polyvinylique, du polyéthylène glycol, de l'acide hyaluronique, de dextrane et de dérivés ou d'analogues de ceux-ci.

9. Stratifié de membrane amniotique de l'une quelconque des revendications 5 à 8, ledit stratifié étant sous la forme d'une feuille, d'un tube ou d'une microsphère.
